# EUROPÄISCHE PATENTSCHRIFT

(11) **EP 2 051 973 B1**
(45) Veröffentlichungstag und Bekanntmachung des Hinweises auf die Patenterteilung: **10.04.2013**
(21) Anmeldenummer: 07786193.8
(22) Anmeldetag: 19.07.2007
(51) Int. Cl.: C07D 309/04, C09K 19/34

(54) **TETRAHYDROPYRANVERBINDUNGEN**
TETRAHYDROPYRAN COMPOUNDS
COMPOSÉS DE TÉTRAHYDROPYRANNE

(30) Priorität: 18.08.2006 DE 102006038949
(43) Veröffentlichungstag der Anmeldung: 29.04.2009
(73) Patentinhaber: Merck Patent GmbH, 64293 Darmstadt (DE)
(72) Erfinder: LIETZAU, Lars, 64295 Darmstadt (DE); CZANTA, Markus, 64298 Darmstadt (DE); MANABE, Atsutaka, 64625 Bensheim (DE)
(86) Internationale Anmeldenummer: PCT/EP2007/006426
(87) Internationale Veröffentlichungsnummer: WO 2008/019746

(56) Entgegenhaltungen:
- EP-A- 1 813 662
- EP-A- 1 842 894
- WO-A-2005/081215
- DE-A1-102004 056 901
- DE-A1-102004 058 002
- US-B2- 7 022 865

## Beschreibung

Die vorliegende Erfindung betrifft Verbindungen mit einem Pyranring, ein flüssigkristallines Medium, dessen Verwendung für elektrooptische Zwecke und dieses Medium enthaltende Anzeigen.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener chemischer Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen, flüssigkristallinen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Tetrahydropyranverbindungen als flüssigkristalline Verbindungen sind bekannt, z. B. aus den Druckschriften WO 2004/106459 und WO 2004/048501. In der Druckschrift DE 102004058002 A1 wird eine Verbindung mit vier Ringen der Formel 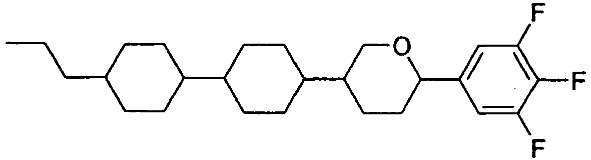 als Bestandteil einer flüssigkristallinen Mischung offenbart.

Gegenstand der Erfindung sind mesogene Verbindungen der Formel I, 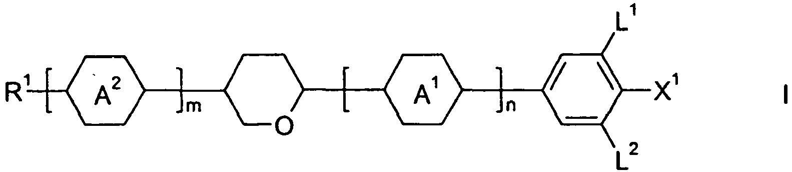 worin
- R¹ und X¹: H, einen unsubstituierten oder mit Halogen einfach oder mehrfach substituierten Alkyl- oder Alkoxyrest mit 1 bis 15 Kohlenstoffatomen oder Alkenyl- oder Alkenyloxyrest mit 2 bis 15 Kohlenstoffatomen bedeutet, wobei in diesen Resten auch eine oder mehrere CH₂-Gruppen jeweils unabhängig voneinander durch -C≡C-, -CH=CH-, -CF=CF-, -O-, -(CO)O- oder -O(CO)- so ersetzt sein können, dass O-Atome nicht direkt miteinander verknüpft sind; oder einer der Reste R¹ und X¹ auch
F, Cl, CN, NCS, SF₅;
- Ring A¹ und A²: 1,4-Cyclohexylen, 1,4-Cyclohexenylen oder 1,4-Cyclohexadienylen, welche unsubstituiert oder durch 1 bis 3 F, bevorzugt einfach, substituiert sind,
- L¹ und L²: unabhängig voneinander H oder F;
- m: 0 oder 1; und
- n: 1, 2 oder 3;
bedeuten, wobei
- m+n: ≥ 2 ist.

Die Verbindungen besitzen demnach 4, 5 oder 6 Ringe. Bevorzugt bedeuten R¹ und X¹ nicht gleichzeitig H.

Die Erfindung betrifft weiterhin die Verwendung dieser Verbindungen als Komponenten flüssigkristalliner Medien. Ein weiterer Gegenstand der Erfindung ist ein flüssigkristallines Medium mit wenigstens zwei flüssigkristallinen Verbindungen, das dadurch gekennzeichnet ist, dass es wenigstens eine erfindungsgemäße Verbindung der Formel I enthält, sowie Flüssigkristall- und elektrooptische Anzeigeelemente, die ein erfindungsgemäßes flüssigkristallines Medium enthalten.

Die Verbindungen der Formel I können als Komponenten flüssigkristalliner, Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag als eine Aufgabe zugrunde, neue stabile, Verbindungen aufzufinden, die als Komponente(n) flüssigkristalliner Medien, insbesondere für TN-, STN-, IPS- und für weitere Aktivmatrix-Displays, geeignet sind.

Eine weitere Aufgabe der vorliegenden Erfindung war es, Verbindungen bereitzustellen, die eine hohe dielektrische Anisotropie Δε, einen hohen Klärpunkt, eine geringe optische Anisotropie sowie eine niedrige Rotationsviskosität γ₁ aufweisen. Darüber hinaus sollten die erfindungsgemäßen Verbindungen thermisch und photochemisch stabil sein. Ferner sollten die erfindungsgemäßen Verbindungen in flüssigkristallinen Mischungen einsetzbar sein, indem sie sich in gebräuchlichen Mischungen lösen lassen und deren flüssigkristalline Phasenbereiche nicht beeinträchtigen oder verbessern.

Es wurde gefunden, dass die erfindungsgemäßen Tetrahydropyranderivate vorzüglich als Komponenten flüssigkristalliner Medien geeignet sind. Mit ihrer Hilfe lassen sich stabile, flüssigkristalline Medien, insbesondere geeignet für TN-TFT, STN und IPS Flüssigkristallanzeigen, erhalten. Die erfindungsgemäßen Verbindungen sind chemisch, thermisch und gegen (UV-)Licht stabil. Sie sind in reinem Zustand farblos. Auch zeichnen sie sich durch eine positive dielektrische Anisotropie Δε aus, aufgrund derer in der Anwendung in optischen Schaltelementen niedrigere Schwellenspannungen erforderlich sind. Darüber hinaus weisen die Verbindungen günstige, d. h. niedrige Werte für die Rotationsviskosität auf. Weitere Vorteile der erfindungsgemäßen Verbindungen sind die höhere Polarität des Tetrahydropyranrings gegenüber Cyclohexanderivaten, wobei kein schädlicher Einfluss auf die (elektro-)optischen Eigenschaften auftritt.

Flüssigkristalline Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt. In diesem Zusammenhang ist es auch möglich, erfindungsgemäße flüssigkristalline Mischungen mit den erfindungsgemäßen Derivaten mit sehr kleinen Werten der optischen Anisotropie und mit gering positiven bis stark positiven Werten der dielektrischen Anisotropie zu erhalten.

Mit der Bereitstellung der erfindungsgemäßen Tetrahydropyranderivate wird ganz allgemein die Palette der Verbindungen, die sich unter verschiedenen, anwendungstechnischen Gesichtspunkten zur Herstellung flüssigkristalliner Mischungen eignen, erheblich verbreitert.

Die erfindungsgemäßen Verbindungen bilden in der Mischung mit geeigneten Cokomponenten flüssigkristalline Mesophasen in einem für die elektrooptische Anwendung günstig gelegenen Temperaturbereich. Flüssigkristalline Medien mit breiten nematischen Phasenbereichen lassen sich aus den erfindungsgemäßen Verbindungen und weiteren Substanzen herstellen. Die erfindungsgemäßen Verbindungen können erfolgreich eingesetzt werden, weil sie in den üblichen Basismischungen gut löslich sind.

Die Tetrahydropyranderivate besitzen einen breiten Anwendungsbereich. In Abhängigkeit von der Auswahl der Substituenten können diese Verbindungen als Basismaterialien dienen, aus denen flüssigkristalline Medien zum großen Teil zusammengesetzt sind. Es können aber auch den erfindungsgemäßen Verbindungen flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Es ist bevorzugt, dass R¹ in den erfindungsgemäßen Verbindungen der Formel I einen geradkettigen Alkyl- oder Alkenylrest, insbesondere einen geradkettigen und unsubstituierten Alkyl- oder Alkenylrest mit 1, 2, 3, 4, 5, 6 oder 7 beziehungsweise 2, 3, 4, 5, 6 oder 7 Kohlenstoffatomen darstellt. Beispielhafte bevorzugte Reste R¹ sind unter anderem Methyl, Ethyl, n-Propyl, n-Butyl, n-Pentyl, n-Hexyl, n-Heptyl, Vinyl, 1 E-Propenyl, 2-Propenyl, 1 E-Butenyl, 3-Butenyl, 1E-Pentenyl, 3E-Pentenyl, 1 E-Hexenyl und 1E-Heptenyl. Der Substituent R¹ ist daher bevorzugt ein geradkettiger Alkyl oder Alkenylrest mit 1-6 bzw. 2-6 C-Atomen.

X¹ bedeutet vorzugsweise F, Cl, CN, NCS, SF₅, einen halogenierten Alkylrest, halogenierten Alkoxyrest, halogenierten Alkenylrest oder halogenierten Alkenyloxyrest mit jeweils bis zu 7 C-Atomen.

X¹ bedeutet besonders bevorzugt F, Cl, CN, CF₃, CF₂H, OCF₃, OCF₂H, OCFHCF₃, OCFHCH₂F, OCFHC₂HF, OCF₂CH₃, OCF₂CH₂F, OCF₂CHF₂, OCF₂CF₂CF₂H, OCF₂CF₂CH₂F, OCFHCF₂CF₃, OCFHCF₂CHF₂, OCFHCFHCF₃, OCH₂CF₂CF₃, OCF₂CF₂CF₃, OCF₂CFHCHF₂, OCF₂CH₂CHF₂, OCFHCF₂CHF₂, OCFHCFHCHF₂, OCFHCH₂CF₃, OCH₂CFHCF₃, OCH₂CF₂CHF₂, OCF₂CFHCH₃, OCF₂CH₂CHF₂, OCFHCF₂CH₃, OCFHCFHCHF₂, OCFHCH₂CF₃, OCH₂CF₂CHF₂, OCH₂CFHCHF₂, OCF₂CH₂CH₃, OCFHCFHCH₃, OCFHCH₂CHF₂, OCH₂CF₂CH₃, OCH₂CFHCHF₂, OCH₂CH₂CHF₂, OCHCH₂CH₃, OCH₂CFHCH₃, OCH₂CH₂CHF₂, OCClFCF₃, OCClFCClF₂, OCClFCHF₂, OCFHCCl₂F, OCClFCHF₂, OCClFCClF₂, OCF₂CHCl₂, OCF₂CHCl₂, OCF₂CCl₂F, OCF₂CClFH, OCF₂CClF₂, OCF₂CF₂CClF₂, OCF₂CF₂CCl₂F, OCClFCF₂CF₃, OCClFCF₂CHF₂, OCClFCF₂CClF₂, OCClFCFHCF₃, OCClFCClFCF₃, OCCl₂CF₂CF₃, OCClHCF₂CF₃, OCClFCF₂CF₃, OCClFCClFCF₃, OCF₂CClFCHF₂, OCF₂CF₂CCl₂F, OCF₂CCl₂CHF₂, OCF₂CH₂CClF₂, OCClFCF₂CFH₂, OCFHCF₂CCl₂F, OCClFCFHCHF₂, OCClFCClFCF₂H, OCFHCFHCClF₂, OCClFCH₂CF₃, OCFHCCl₂CF₃, OCCl₂CFHCF₃, OCH₂CClFCF₃, OCCl₂CF₂CF₂H, OCH₂CF₂CClF₂, OCF₂CClFCH₃, OCF₂CFHCCl₂H, OCF₂CCl₂CFH₂, OCF₂CH₂CCl₂F, OCClFCF₂CH₃, OCFHCF₂CCl₂H, OCClFCClFCHF₂, OCFHCFHCCl₂F, OCClFCH₂CF₃, OCFHCCl₂CF₃, OCCl₂CF₂CFH₂, OCH₂CF₂CCl₂F, OCCl₂CFHCF₂H, OCClHCClFCF₂H, OCF₂CClHCClH₂, OCF₂CH₂CCl₂H, OCClFCFHCH₃, OCF₂CClFCCl₂H, OCClFCH₂CFH₂, OCFHCCl₂CFH₂, OCCl₂CF₂CH₃, OCH₂CF₂CClH₂, OCCl₂CFHCFH₂, OCH₂CClFCFCl₂, OCH₂CH₂CF₂H, OCClHCClHCF₂H, OCH₂CCl₂CF₂H, OCClFCH₂CH₃, OCFHCH₂CCl₂H, OCClHCFHCClH₂, OCH₂CFHCCl₂H, OCCl₂CH₂CF₂H, OCH₂CCl₂CF₂H, CH=CF₂, OCH=CF₂, CF=CF₂, OCF=CF₂, CF=CHF, OCF=CHF, CH=CHF, OCH=CHF insbesondere F, Cl, CN, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CHF₂.

Ganz besonders ist es bevorzugt, dass X¹ in den erfindungsgemäßen Verbindungen der Formel I eine Gruppe aus F, Cl, CF₃, OCF₃, OCHF₂ oder CN bedeutet, darunter insbesondere F oder OCF₃.

Die Ringe A¹ und A² sind bevorzugt 1,4-Cyclohexylen oder 1,4-Cyclohexenylen, wahlweise mit einem Fluoratom substituiert, insbesondere 1,4-Cyclohexylen, welches besonders bevorzugt nicht substituiert ist.

Die 1,4-Cyclohexenylengruppe ist bevorzugt der Formel

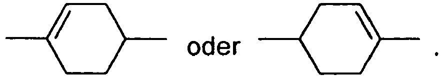

Für den Fall, dass einer der Ringe A¹ und A² mit einem Fluoratom substituiert ist, so ist er bevorzugt der Formel 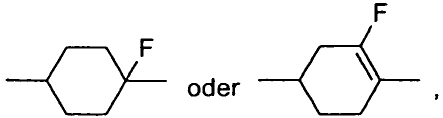 insbesondere der letzteren Formel.

Die bevorzugten Werte für m und n sind m = 0 und n = 2 oder m = 1 und n = 1. Daher ist die Summe aus m und n bevorzugt 2.

Bevorzugte Ausführungsformen der erfindungsgemäßen Verbindungen der Formel I sind ausgewählt aus Verbindungen der Formeln I1 bis I30: 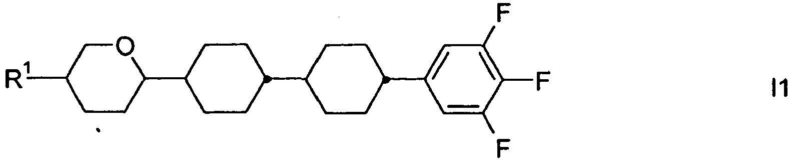 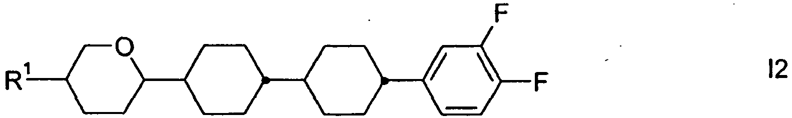 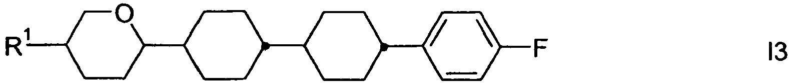 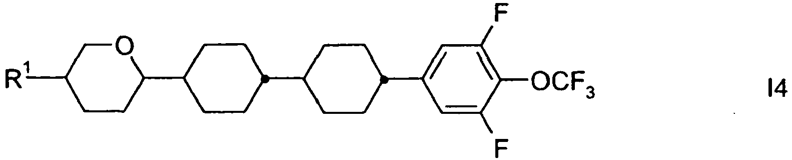 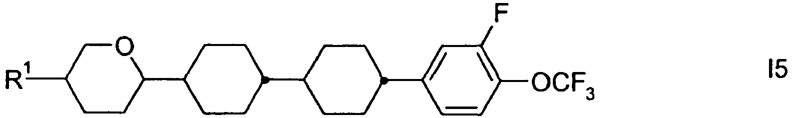 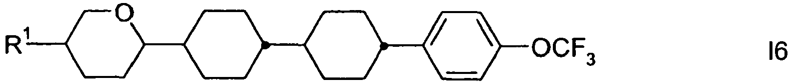 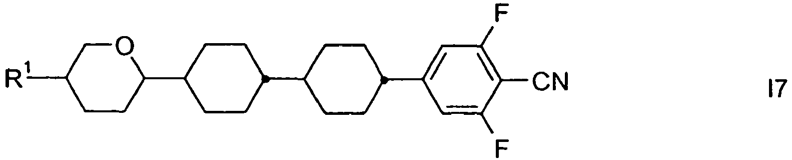 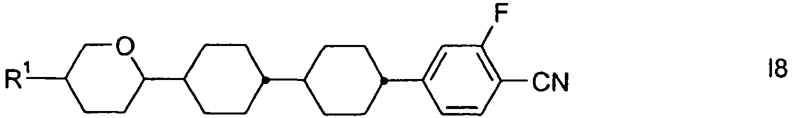 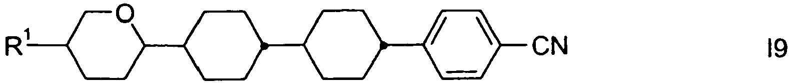 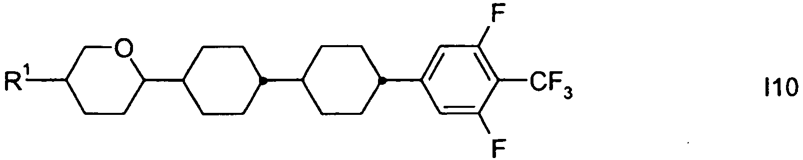 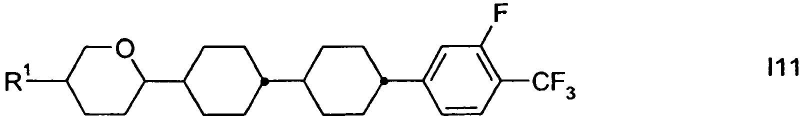 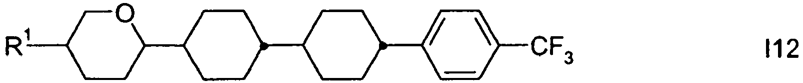 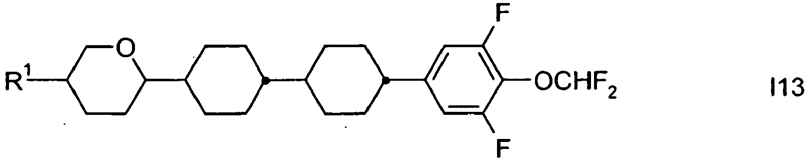 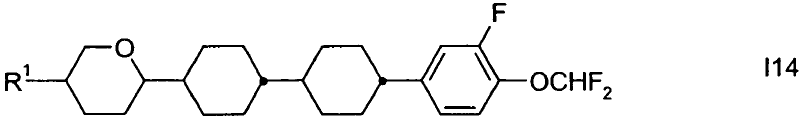 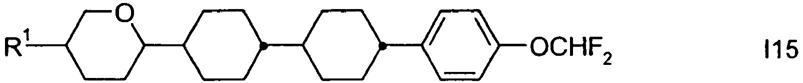 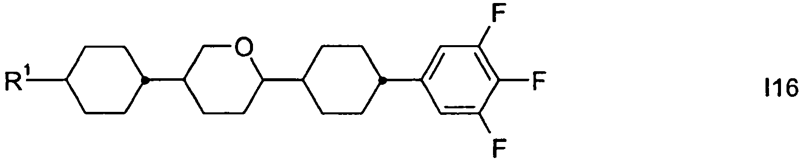 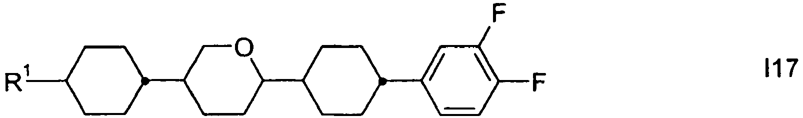 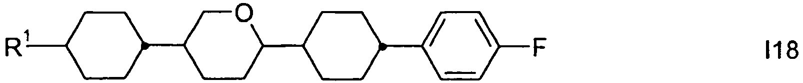 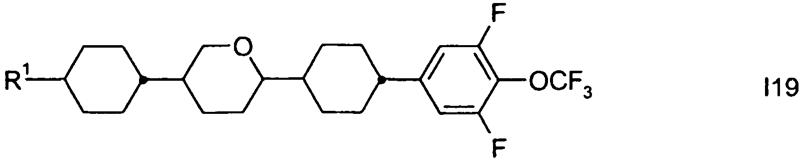 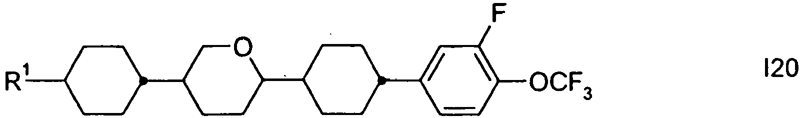 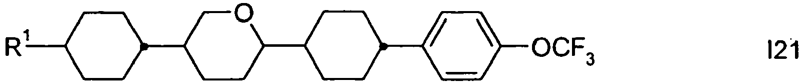 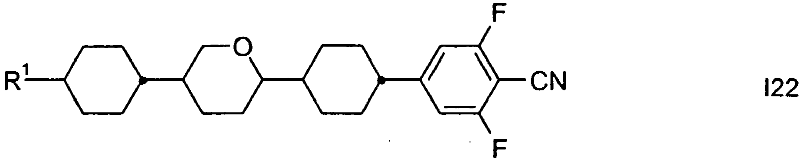 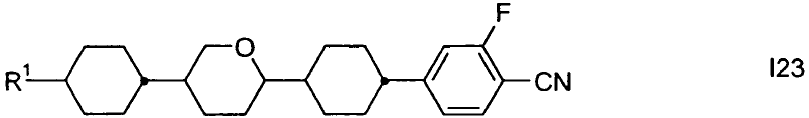 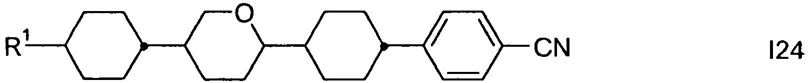 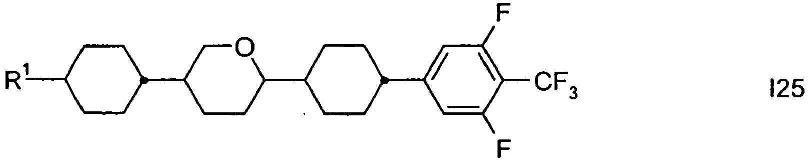 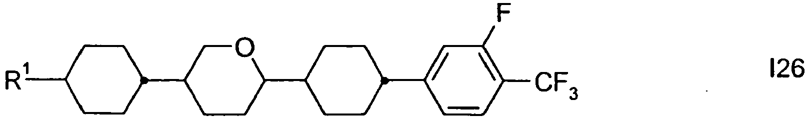 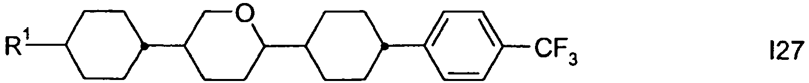 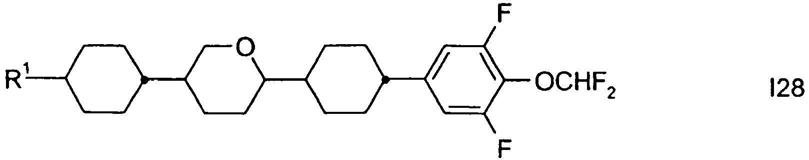 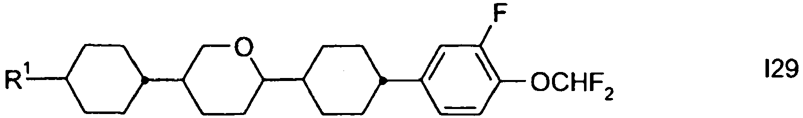 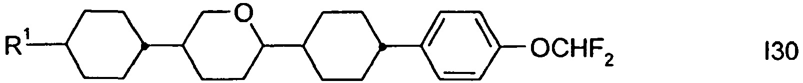 wobei R¹ wie oben für Formel I definiert ist oder bevorzugt einen geradkettigen Alkyl- oder Alkenylrest, insbesondere einen geradkettigen und unsubstituierten Alkyl- oder Alkenylrest mit 1 (nur für Alkyl), 2, 3, 4, 5, 6 oder 7 Kohlenstoffatomen darstellt. Besonders bevorzugte Verbindungen sind die Verbindungen der Formeln I1, I4, I16 und I19, insbesondere der Formeln 11 und I19.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen. Entsprechend sind auch die erfindungsgemäßen Verbindungen der Formel I durch andere als dem erfindungsgemäßen Verfahren zugänglich.

Besonders geeignet zur Herstellung sind Synthesemethoden gemäß Schema 1, 2, 3, 4 und 5. Schema 1 und 2 geben zusammen ein Herstellungsverfahren der erfindungsgemäßen Verbindungen der Formel I mit m = 0 und n = 2 wieder.

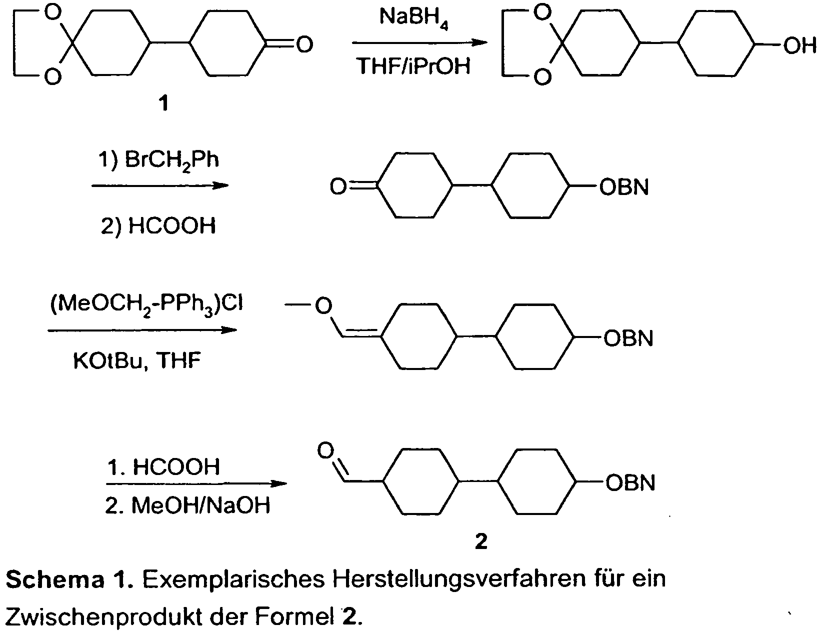

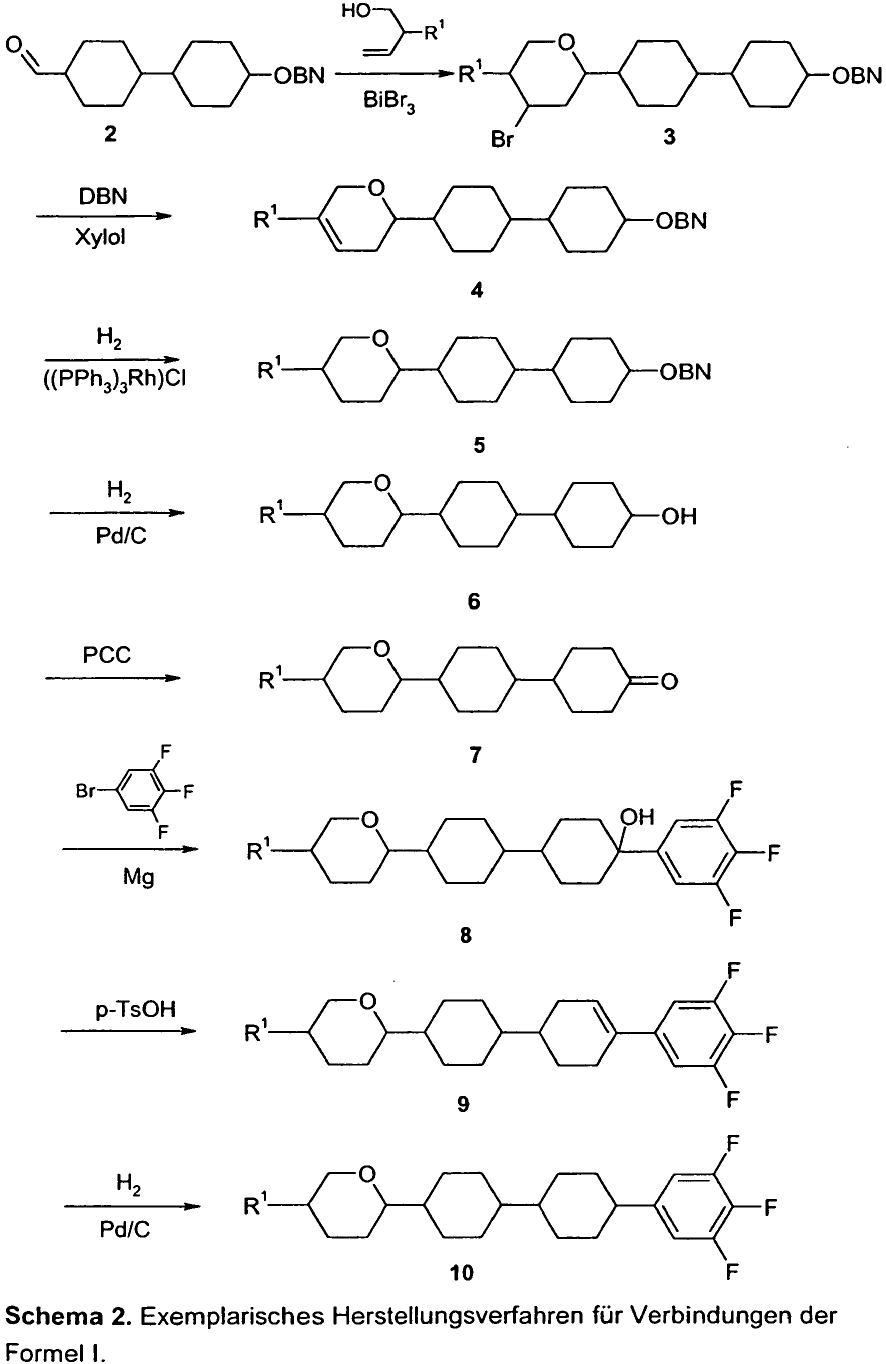

Über den Synthesebaustein 11 werden auch ungesättigte Seitenketten in die Moleküle eingeführt (Schema 3).

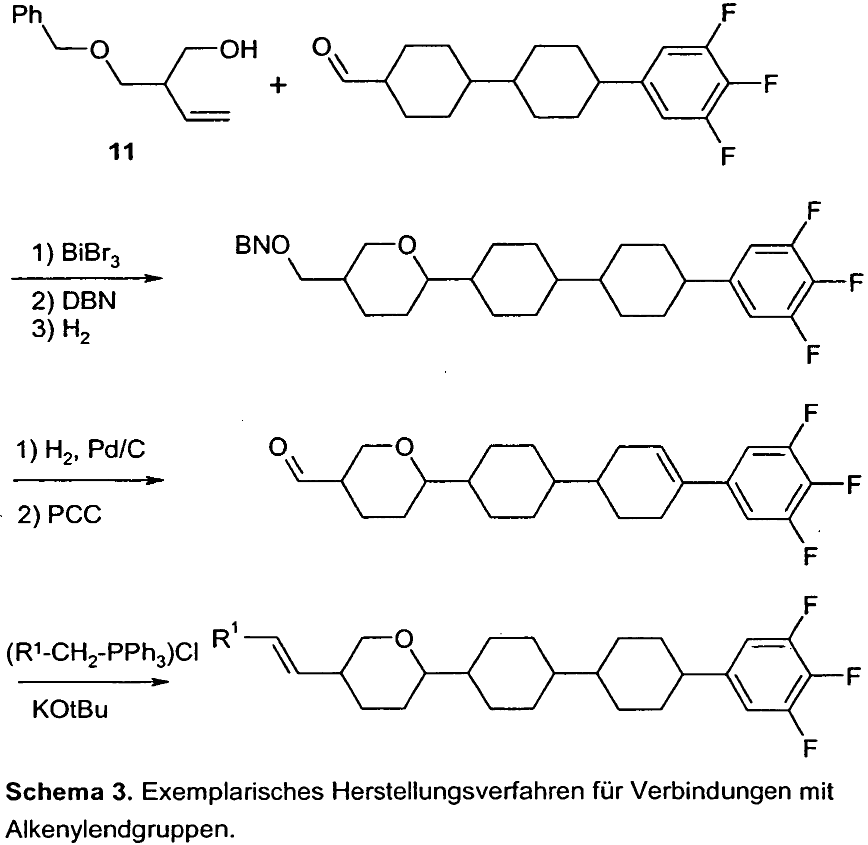

Die Benzyloxygruppe der Verbindung 11 kann in den nachfolgenden Reaktionsstufen gemäß Schema 3 in eine Alkenylgruppe umgewandelt werden, indem der Benzylrest reduktiv abgespalten wird und die freie Hydroxygruppe zum Aldehyd oxidiert wird (analog Beispiel 1; Reaktionsschritte 1.4 und 1.5). Anschließend wird an Stelle der Carbonylgruppe des Aldehyds durch eine Wittig-Horner-Reaktion ein wahlweise substituiertes Alken erzeugt.

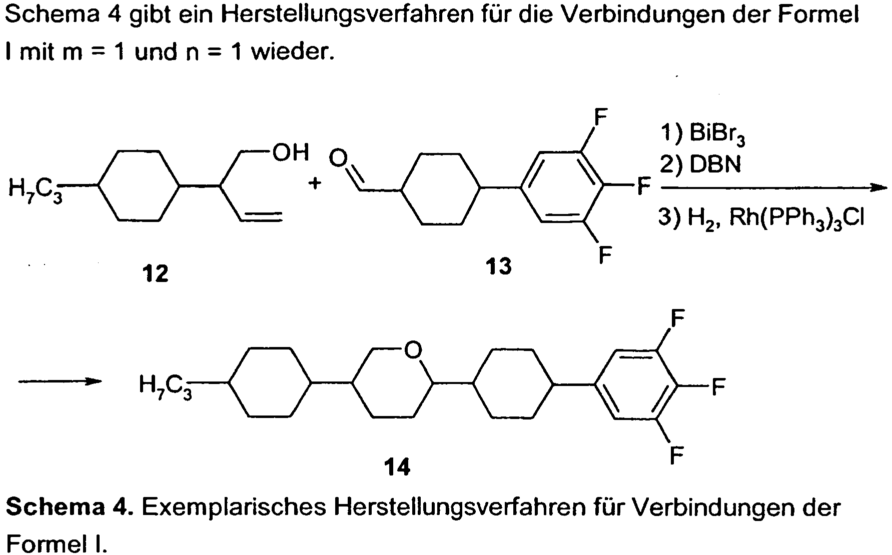

Über den Synthesebaustein 15 werden ungesättigte Seitenketten in die Moleküle eingeführt:

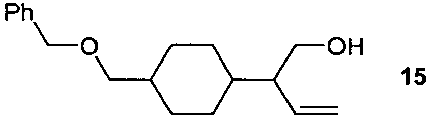

Der Synthesebaustein 15 ist ein benzyloxysubstituiertes Analogon zu der Verbindung 12 (vgl. Schema 4). Nach der Reaktion gemäß Schema 4 kann die Benzyloxy-Gruppe gemäß Schema 3 in eine Alkenylgruppe umgewandelt werden.

Aus geeigneten Ausgangsverbindungen kann eine Verbindung mit der Ringabfolge der Formel 14 auch über eine Addition einer Arylmetallverbindung (aus 17) an ein entsprechend substituiertes tricyclisches Cyclohexanon der Formel 16 hergestellt werden (Schema 5).

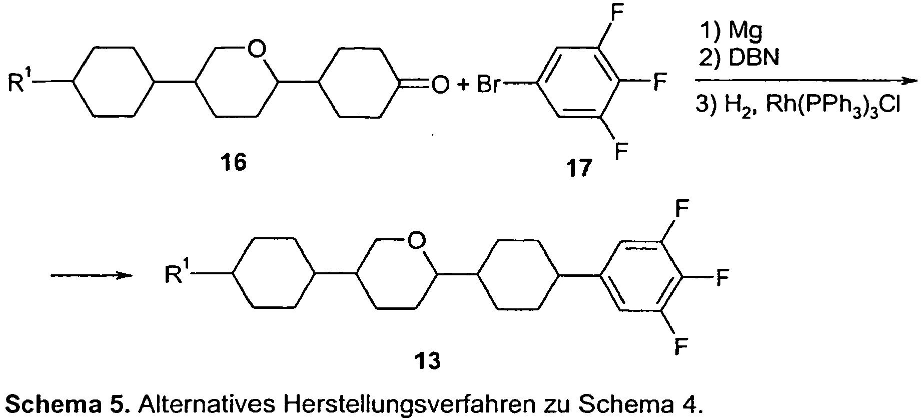

Die Erfindung umfasst auch ein Verfahren zur Herstellung von Verbindungen der Formel I umfassend einen Verfahrensschritt, der dadurch gekennzeichnet ist, dass ein Cyclohexanon der Formel II 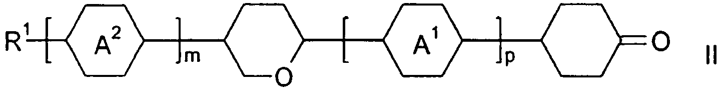 worin
R¹, A¹, A² und m wie in Formel I definiert sind, und
p 0, 1 oder 2 bedeutet,
mit einer Arylmetallverbindung der Formel III 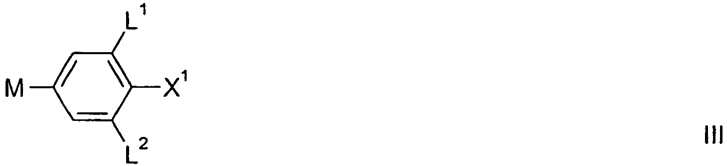 worin
L¹, L² und X¹ wie in Formel I definiert sind, und
M Li, MgCl oder MgBr bedeutet,
umsetzt.

Sofern Reste oder Substituenten der erfindungsgemäßen Verbindungen beziehungsweise die erfindungsgemäßen Verbindungen selbst als optisch aktive Reste, Substituenten beziehungsweise Verbindungen vorliegen können, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der Formel I in isomerenreiner Form, zum Beispiel als reine Enantiomeren oder Diastereomeren, oder als Gemisch mehrerer Isomeren, zum Beispiel als Racemat, vorliegen können.

Sofern R¹ in Formel I nicht H bedeutet, können die erfindungsgemäßen Verbindungen der Formel I aufgrund der Disubstitution des Pyranrings sowohl als cis- als auch als trans-Isomeren vorliegen. Im allgemeinen ist für viele Verwendungen das jeweilige trans-Isomer bevorzugt. Es kann unter anderem dadurch selektiv erhalten werden, dass im Herstellungsverfahren eine Vorstufe mit Pyranring mit trans-Konfiguration eingesetzt wird, die ihrerseits zum Beispiel durch Isomerisierung mit Base oder Säure, eine Herstellung analog Schema 2, durch Umkristallisation und/oder durch chromatographische Trennung erhalten wird. Diese üblichen Verfahren können natürlich auch mit Isomerengemischen der Endverbindungen der Formel I durchgeführt werden.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten. Man erhält sie vorzugsweise indem man die Komponenten miteinander vermischt. Vorzugsweise basieren sie auf mehreren (vorzugsweise zwei, drei oder mehr) Verbindungen der Formel I. Der Anteil der Verbindungen der Formel I ist im allgemeinen 1-95 %, vorzugsweise 2-60 % und besonders bevorzugt im Bereich von 5-30 %. Ein erfindungsgemäßes Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-CF₂O-E-R" 3

R-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Py-, -G-Phe- und -G-Cyc- sowie aus deren Spiegelbildern gebildeten Gruppe, wobei Phe unsubstituiertes oder durch Fluor substituiertes 1,4-Phenylen, Cyc trans-1,4-Cyclohexylen, Pyr Pyrimidin-2,5-diyl oder Pyridin-2,5-diyl, Dio 1,3-Dioxan-2,5-diyl, Py Tetrahydropyran-2,5-diyl- und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS oder -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢCH₃₋ₖFₖ, wobei i 0 oder 1 und k 1, 2 oder 3 ist. Die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS; -CF₃, -OCHF₂ oder -OCF₃ hat.

In den Verbindungen der Teilformeln 1b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:

| | |
|---|---|
| Gruppe A: | 0 bis 90 %, vorzugsweise 20 bis 90 %, besonders bevorzugt 30 bis 90 %; |
| Gruppe B: | 0 bis 80 %, vorzugsweise 10 bis 80 %, besonders bevorzugt 10 bis 65 %; |
| Gruppe C: | 0 bis 80 %, vorzugsweise 0 bis 80 %, besonders bevorzugt 0 bis 50 %; |

wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben.

Die erfindungsgemäßen Flüssigkristallmischungen ermöglichen eine bedeutende Erweiterung des zur Verfügung stehenden Parameterraumes.

Gegenstand der Erfindung sind auch elektrooptische Anzeigen (insbesondere TFT-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Der Ausdruck "Alkyl" umfasst geradkettige und verzweigte Alkylgruppen mit 1-9 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im Allgemeinen bevorzugt.

Der Ausdruck "Alkenyl" umfasst geradkettige und verzweigte Alkenylgruppen mit bis zu 9 Kohlenstoffatomen, insbesondere die geradkettigen Gruppen. Besonders bevorzugte Alkenylgruppen sind C₂₋C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₃-C₇-4-Alkenyl. Beispiele bevorzugter Alkenylgruppen sind Vinyl, 1E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃. Der Ausdruck "halogenierte Alkenylreste" und verwandte Ausdrücke erklären sich entsprechend.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften.

Der Aufbau der erfindungsgemäßen Matrix-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der Matrix-Anzeige, insbesondere auch Matrix-Anzeigeelemente auf Basis von poly-Si TFT.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Weitere Kombinationen der Ausführungsformen und Variationen der Erfindungsgegenstände sind in den Patentansprüchen offenbart.

Die folgenden Beispiele sollen die Erfindung erläutern.

Vor- und nachstehend bedeuten Prozentangaben Gewichts-prozent. Alle Temperaturen sind in Grad Celsius angegeben. Ferner bedeuten K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1 kHz, 20 °C) und γ₁ die Rotationsviskosität (in der Einheit mPa·s).

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.

Die dielektrische Anisotropie Δε der einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100 % extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation.

Folgende Abkürzungen werden in den Beispielen sowie in den Synthese- und Reaktionsschemata verwendet:
- DBN: Diazabicycloundecen
- MTB-Ether: Methyl-*tert*-butylether
- OBN: O-Benzyl-Gruppe
- n-BuLi: 1,6 molare Lösung von n-Butyllithium in n-Hexan
- THF: Tetrahydrofuran
- p-TsOH: *p*-Toluolsulfonsäure
- RT: Raumtemperatur
- Pd/C: Palladium auf Aktivkohle (5 %), wasserhaltig
- KOtBu: Kalium-*tert*-butoxid

### Beispiel 1

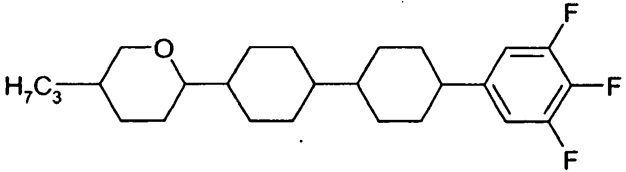

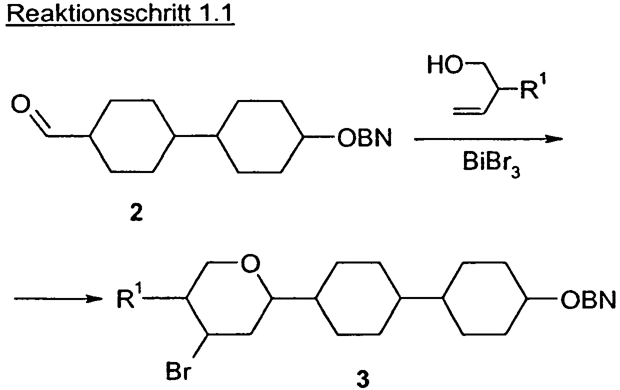

30,0 g (100 mmol) des Aldehyds 2 und 19,0 g (60 %ig; 100 mmol) 2-Vinylpropanol werden in 140 ml Dichlormethan gelöst und mit 22,9 g (50 mmol) Bismut(III)bromid versetzt. Der Ansatz wird über Nacht bei RT gerührt. Anschließend wird der Ansatz über Kieselgel filtriert und eingeengt. Es wird die Bromverbindung 3 isoliert.

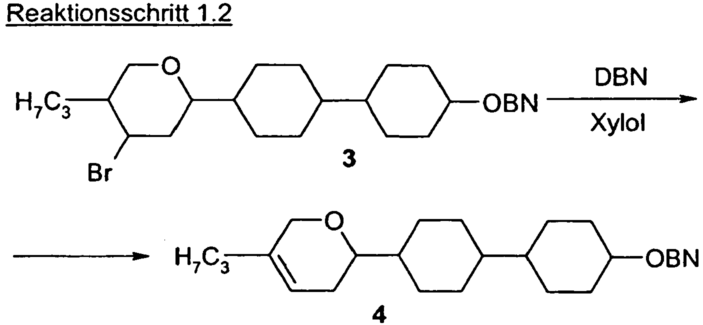

Unter Stickstoff werden 53 g (111 mmol) der Bromverbindung 3 in 90 ml Toluol gelöst, mit 20 ml DBN versetzt und 8 h zum Sieden erhitzt. Anschließend wird der abgekühlte Ansatz mit 400 ml Wasser versetzt und mit verd. Schwefelsäure angesäuert. Die organische Phase wird abgetrennt, mit Natriumhydrogencarbonat-Lösung gewaschen und eingeengt. Der erhaltene Rückstand wird über Kieselgel gegeben (Toluol). Es wird die Verbindung 4 isoliert.

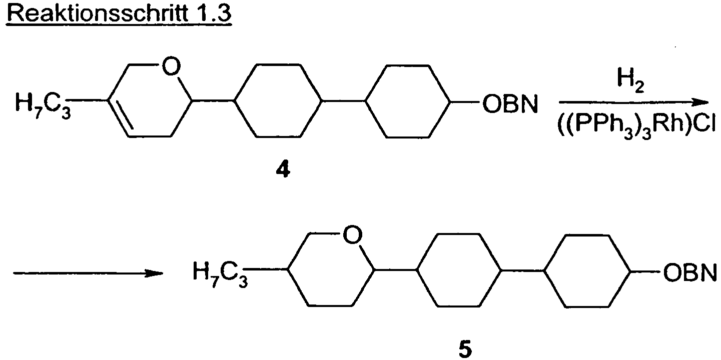

27 g (68 mmol) des Dihydropyrans 4 werden in 280 ml Methanol und 80 ml Toluol gelöst und mittels (PPh₃)₃RhCl-Katalysator (1 %) bei 8 bar/80 °C bis zum Ende der Wasserstoffaufnahme hydriert. Die Hydrierlösung wird eingeengt und der Rückstand über Kieselgel gegeben (Toluol/MTB-Ether 9:1)

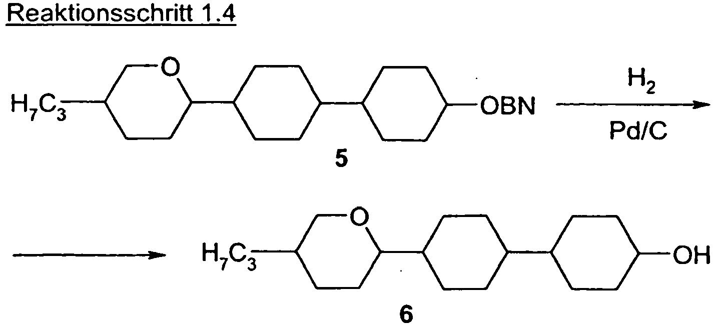

23,5 g (59 mmol) des Benzylethers 5 werden in 250 ml THF gelöst und am Palladium-Katalysator hydriert. Anschließend wird der Katalysator abgetrennt und die Lösung eingeengt. Der erhaltene Rückstand wird ohne weitere Reinigung in der folgenden Stufe eingesetzt.

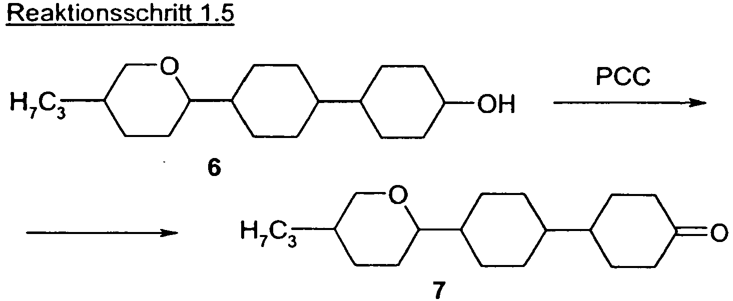

Unter Stickstoff wird eine Suspension von 200 g Kieselgur in 2,5 I Dichlormethan zunächst mit 102 g (470 mmol) Pyridiniumchlorochromat (PCC) und anschließend mit 138,6 g (449 mmol) des Alkohols 6 versetzt. Die Suspension wird 16 h bei RT gerührt und über Celite abgesaugt. Das Filtrat wird eingeengt, und der Rückstand wird über Kieselgel gegeben (Toluol/MTB-Ether 9:1).

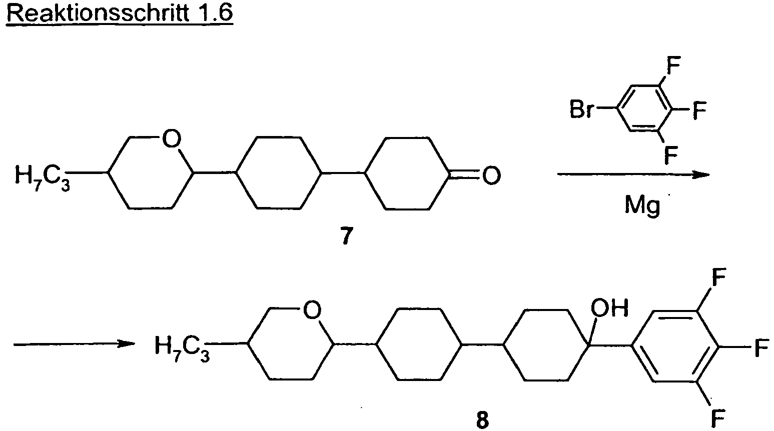

1,9 g (78 mmol) Magnesium werden mit 9,3 ml (78 mmol) Trifluorbrombenzol gelöst in 30 ml THF versetzt und die entsprechende Grignard-Verbindung hergestellt. In der Siedehitze wird eine Lösung des Ketons 7 in 30 ml THF zugegeben und 1 h unter Rückfluss erhitzt. Anschließend wird der Ansatz mit Wasser hydrolysiert, mit Salzsäure auf pH 1 eingestellt und mit 200 ml MTB-Ether versetzt. Die organische Phase wird eingeengt, und das Produkt ohne weitere Reinigung in der nächsten Stufe umgesetzt.

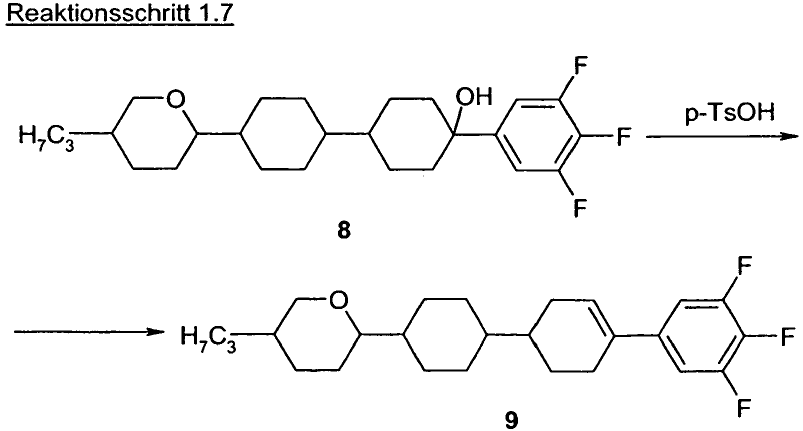

Der Alkohol 8 (ca. 20 mmol) wird mit 300 ml Xylol und 500 mg p-Toluolsulfonsäure versetzt und 30 min am Wasserabscheider unter Rückfluss erhitzt. Anschließend wird der Ansatz über Kieselgel gegeben (Toluol) und eingeengt. Der Rückstand wird ohne weitere Reinigung umgesetzt.

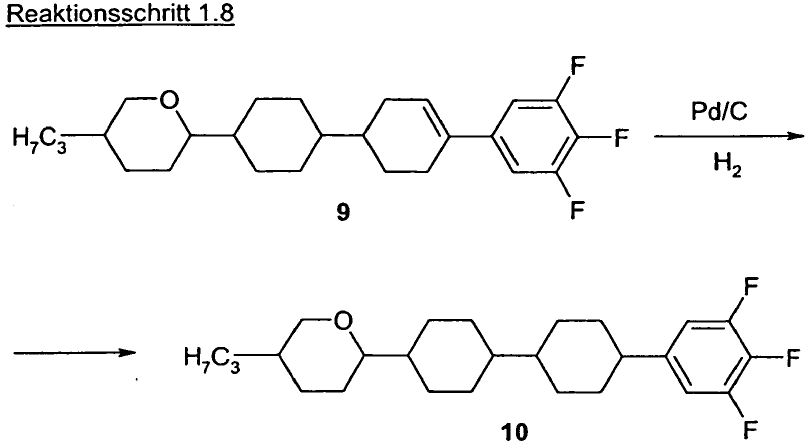

Die Cyclohexen-Verbindung 9 (4,9 g; 12 mmol) wird in 50 ml THF gelöst und am Palladium-Katalysator hydriert. Die Hydrierlösung wird eingeengt und der Rückstand über Kieselgel gegeben und anschließend aus Heptan kristallisiert.
K 95 SmB 169 N 246 I
Δε 14
Δn 0,106

Analog zu Reaktionsschritt 1.1 bis 1.8 werden die folgenden Verbindungen der Formel 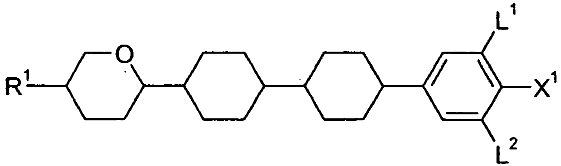 hergestellt:

| R¹ | X¹ | L¹ | L² | Werte |
|---|---|---|---|---|
| H | F | H | H | |
| CH₃ | F | H | H | |
| C₂H₅ | F | H | H | |
| n-C₃H₇ | F | H | H | K 77 SmB 253 N 304 I; Δε 7,0; Δn 0,113 |
| n-C₄H₉ | F | H | H | |
| n-C₅H₁₁ | F | H | H | |
| n-C₆H₁₃ | F | H | H | |
| H | F | F | H | |
| CH₃ | F | F | H | |
| C₂H₅ | F | F | H | |
| n-C₃H₇ | F | F | H | K 35 SmB 215 N 278 I; Δε 10; Δn 0,107 |
| n-C₄H₉ | F | F | H | |
| n-C₅H₁₁ | F | F | H | |
| n-C₆H₁₃ | F | F | H | |
| H | F | F | F | |
| CH₃ | F | F | F | |
| C₂H₅ | F | F | F | |
| n-C₃H₇ | F | F | F | (vgl. Beispiel 1) |
| nC₄H₉ | F | F | F | |
| n-C₅H₁₁ | F | F | F | |
| n-C₆H₁₃ | F | F | F | |
| H | OCF₃ | H | H | |
| CH₃ | OCF₃ | H | H | |
| C₂H₅ | OCF₃ | H | H | |
| n-C₃H₇ | OCF₃ | H | H | |
| n-C₄H₉ | OCF₃ | H | H | |
| n-C₅H₁₁ | OCF₃ | H | H | |
| n-C₆H₁₃ | OCF₃ | H | H | |
| H | OCF₃ | F | H | |
| CH₃ | OCF₃ | F | H | |
| C₂H₅ | OCF₃ | F | H | |
| n-C₃H₇ | OCF₃ | F | H | |
| n-C₄H₉ | OCF₃ | F | H | |
| n-C₅H₁₁ | OCF₃ | F | H | |
| n-C₆H₁₃ | OCF₃ | F | H | |
| H | OCF₃ | F | F | |
| CH₃ | OCF₃ | F | F | |
| C₂H₅ | OCF₃ | F | F | |
| n-C₃H₇ | OCF₃ | F | F | K 78 SmB(C) 147 SmB(H) 167 N 264 I; Δε 15; Δn 0,104 |
| nC₄H₉ | OCF₃ | F | F | |
| n-C₅H₁₁ | OCF₃ | F | F | |
| n-C₆H₁₃ | OCF₃ | F | F | |
| H | CN | H | H | |
| CH₃ | CN | H | H | |
| C₂H₅ | CN | H | H | |
| n-C₃H₇ | CN | H | H | |
| n-C₄H₉ | CN | H | H | |
| n-C₅H₁₁ | CN | H | H | |
| n-C₆H₁₃ | CN | H | H | |
| H | CN | F | H | |
| CH₃ | CN | F | H | |
| C₂H₅ | CN | F | H | |
| n-C₃H₇ | CN | F | H | |
| n-C₄H₉ | CN | F | H | |
| n-C₅H₁₁ | CN | F | H | |
| n-C₆H₁₃ | CN | F | H | |
| H | CN | F | F | |
| CH₃ | CN | F | F | |
| C₂H₅ | CN | F | F | |
| n-C₃H₇ | CN | F | F | |
| nC₄H₉ | CN | F | F | |
| n-C₅H₁₁ | CN | F | F | |
| n-C₆H₁₃ | CN | F | F | |
| H | OCHF₂ | H | H | |
| CH₃ | OCHF₂ | H | H | |
| C₂H₅ | OCHF₂ | H | H | |
| n-C₃H₇ | OCHF₂ | H | H | |
| n-C₄H₉ | OCHF₂ | H | H | |
| n-C₅H₁₁ | OCHF₂ | H | H | |
| n-C₆H₁₃ | OCHF₂ | H | H | |
| H | OCHF₂ | F | H | |
| CH₃ | OCHF₂ | F | H | |
| C₂H₅ | OCHF₂ | F | H | |
| n-C₃H₇ | OCHF₂ | F | H | |
| n-C₄H₉ | OCHF₂ | F | H | |
| n-C₅H₁₁ | OCHF₂ | F | H | |
| n-C₆H₁₃ | OCHF₂ | F | H | |
| H | OCHF₂ | F | F | |
| CH₃ | OCHF₂ | F | F | |
| C₂H₅ | OCHF₂ | F | F | |
| n-C₃H₇ | OCHF₂ | F | F | |
| nC₄H₉ | OCHF₂ | F | F | |
| n-C₅H₁₁ | OCHF₂ | F | F | |
| n-C₆H₁₃ | OCHF₂ | F | F | |
| H | CF₃ | H | H | |
| CH₃ | CF₃ | H | H | |
| C₂H₅ | CF₃ | H | H | |
| n-C₃H₇ | CF₃ | H | H | |
| n-C₄H₉ | CF₃ | H | H | |
| n-C₅H₁₁ | CF₃ | H | H | |
| n-C₆H₁₃ | CF₃ | H | H | |
| H | CF₃ | F | H | |
| CH₃ | CF₃ | F | H | |
| C₂H₅ | CF₃ | F | H | |
| n-C₃H₇ | CF₃ | F | H | |
| n-C₄H₉ | CF₃ | F | H | |
| n-C₅H₁₁ | CF₃ | F | H | |
| n-C₆H₁₃ | CF₃ | F | H | |
| H | CF₃ | F | F | |
| CH₃ | CF₃ | F | F | |
| C₂H₅ | CF₃ | F | F | |
| n-C₃H₇ | CF₃ | F | F | |
| nC₄H₉ | CF₃ | F | F | |
| n-C₅H₁₁ | CF₃ | F | F | |
| n-C₆H₁₃ | CF₃ | F | F | |
| H | CH₃ | F | F | |
| CH₃ | CH₃ | F | F | |
| C₂H₅ | CH₃ | F | F | |
| n-C₃H₇ | CH₃ | F | F | K 72 SmB(C) (-12) SmB(H) 259 N 280 I; Δε 4,6; Δn 0,113 |
| nC₄H₉ | CH₃ | F | F | |
| n-C₅H₁₁ | CH₃ | F | F | |
| n-C₆H₁₃ | CH₃ | F | F | |
| H | CH₃ | F | H | |
| CH₃ | CH₃ | F | H | |
| C₂H₅ | CH₃ | F | H | |
| n-C₃H₇ | CH₃ | F | H | K 63 SmB 276 N 301 I; Δε 2,8; Δn 0,124 |
| nC₄H₉ | CH₃ | F | H | |
| n-C₅H₁₁ | CH₃ | F | H | |
| n-C₆H₁₃ | CH₃ | F | H | |
| H | CH₃ | H | H | |
| CH₃ | CH₃ | H | H | |
| C₂H₅ | CH₃ | H | H | |
| n-C₃H₇ | CH₃ | H | H | |
| nC₄H₉ | CH₃ | H | H | |
| n-C₅H₁₁ | CH₃ | H | H | |
| n-C₆H₁₃ | CH₃ | H | H | |

### Beispiel 2

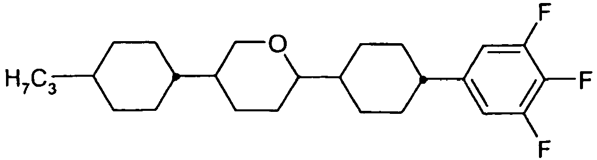

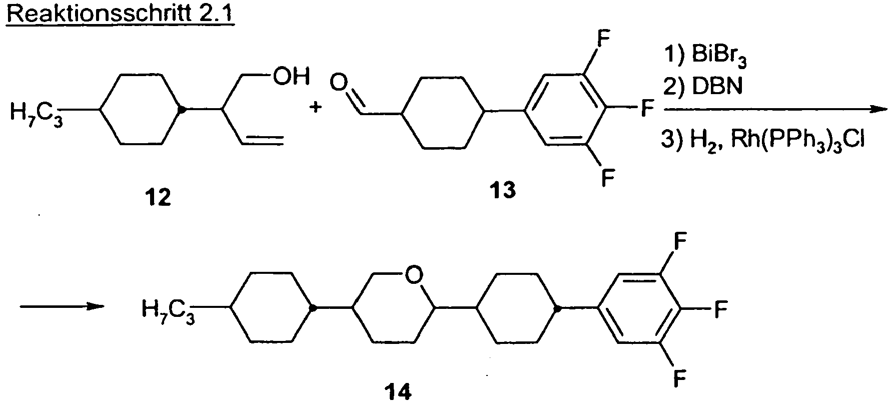

Aus den Zwischenstufen 12 und 13 wird analog zu Reaktionsschritt 1.1 bis 1.3 das Molekül 14 hergestellt.
K 102 SmB 118 N 236 I
Δε 14
Δn 0,098

Analog zu Reaktionsschritt 2.1 werden die folgenden Verbindungen der Formel 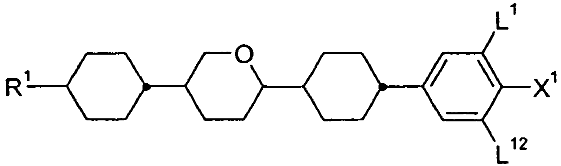 hergestellt:

| R¹ | X¹ | L¹ | L² | Werte |
|---|---|---|---|---|
| H | F | H | H | |
| CH₃ | F | H | H | |
| C₂H₅ | F | H | H | |
| n-C₃H₇ | F | H | H | |
| n-C₄H₉ | F | H | H | |
| n-C₅H₁₁ | F | H | H | |
| n-C₆H₁₃ | F | H | H | |
| H | F | F | H | |
| CH₃ | F | F | H | |
| C₂H₅ | F | F | H | |
| n-C₃H₇ | F | F | H | |
| n-C₄H₉ | F | F | H | |
| n-C₅H₁₁ | F | F | H | |
| n-C₆H₁₃ | F | F | H | |
| H | F | F | F | |
| CH₃ | F | F | F | |
| C₂H₅ | F | F | F | |
| n-C₃H₇ | F | F | F | (vgl. Beispiel 2) |
| nC₄H₉ | F | F | F | |
| n-C₅H₁₁ | F | F | F | |
| n-C₆H₁₃ | F | F | F | |
| H | OCF₃ | H | H | |
| CH₃ | OCF₃ | H | H | |
| C₂H₅ | OCF₃ | H | H | |
| n-C₃H₇ | OCF₃ | H | H | |
| n-C₄H₉ | OCF₃ | H | H | |
| n-C₅H₁₁ | OCF₃ | H | H | |
| n-C₆H₁₃ | OCF₃ | H | H | |
| H | OCF₃ | F | H | |
| CH₃ | OCF₃ | F | H | |
| C₂H₅ | OCF₃ | F | H | |
| n-C₃H₇ | OCF₃ | F | H | |
| n-C₄H₉ | OCF₃ | F | H | |
| n-C₅H₁₁ | OCF₃ | F | H | |
| n-C₆H₁₃ | OCF₃ | F | H | |
| H | OCF₃ | F | F | |
| CH₃ | OCF₃ | F | F | |
| C₂H₅ | OCF₃ | F | F | |
| n-C₃H₇ | OCF₃ | F | F | |
| nC₄H₉ | OCF₃ | F | F | |
| n-C₅H₁₁ | OCF₃ | F | F | |
| n-C₆H₁₃ | OCF₃ | F | F | |
| H | CN | H | H | |
| CH₃ | CN | H | H | |
| C₂H₅ | CN | H | H | |
| n-C₃H₇ | CN | H | H | |
| n-C₄H₉ | CN | H | H | |
| n-C₅H₁₁ | CN | H | H | |
| n-C₆H₁₃ | CN | H | H | |
| H | CN | F | H | |
| CH₃ | CN | F | H | |
| C₂H₅ | CN | F | H | |
| n-C₃H₇ | CN | F | H | |
| n-C₄H₉ | CN | F | H | |
| n-C₅H₁₁ | CN | F | H | |
| n-C₆H₁₃ | CN | F | H | |
| H | CN | F | F | |
| CH₃ | CN | F | F | |
| C₂H₅ | CN | F | F | |
| n-C₃H₇ | CN | F | F | |
| nC₄H₉ | CN | F | F | |
| n-C₅H₁₁ | CN | F | F | |
| n-C₆H₁₃ | CN | F | F | |
| H | OCHF₂ | H | H | |
| CH₃ | OCHF₂ | H | H | |
| C₂H₅ | OCHF₂ | H | H | |
| n-C₃H₇ | OCHF₂ | H | H | |
| n-C₄H₉ | OCHF₂ | H | H | |
| n-C₅H₁₁ | OCHF₂ | H | H | |
| n-C₆H₁₃ | OCHF₂ | H | H | |
| H | OCHF₂ | F | H | |
| CH₃ | OCHF₂ | F | H | |
| C₂H₅ | OCHF₂ | F | H | |
| n-C₃H₇ | OCHF₂ | F | H | |
| n-C₄H₉ | OCHF₂ | F | H | |
| n-C₅H₁₁ | OCHF₂ | F | H | |
| n-C₆H₁₃ | OCHF₂ | F | H | |
| H | OCHF₂ | F | F | |
| CH₃ | OCHF₂ | F | F | |
| C₂H₅ | OCHF₂ | F | F | |
| n-C₃H₇ | OCHF₂ | F | F | |
| nC₄H₉ | OCHF₂ | F | F | |
| n-C₅H₁₁ | OCHF₂ | F | F | |
| n-C₆H₁₃ | OCHF₂ | F | F | |
| H | CF₃ | H | H | |
| CH₃ | CF₃ | H | H | |
| C₂H₅ | CF₃ | H | H | |
| n-C₃H₇ | CF₃ | H | H | |
| n-C₄H₉ | CF₃ | H | H | |
| n-C₅H₁₁ | CF₃ | H | H | |
| n-C₆H₁₃ | CF₃ | H | H | |
| H | CF₃ | F | H | |
| CH₃ | CF₃ | F | H | |
| C₂H₅ | CF₃ | F | H | |
| n-C₃H₇ | CF₃ | F | H | |
| n-C₄H₉ | CF₃ | F | H | |
| n-C₅H₁₁ | CF₃ | F | H | |
| n-C₆H₁₃ | CF₃ | F | H | |
| H | CF₃ | F | F | |
| CH₃ | CF₃ | F | F | |
| C₂H₅ | CF₃ | F | F | |
| n-C₃H₇ | CF₃ | F | F | |
| nC₄H₉ | CF₃ | F | F | |
| n-C₅H₁₁ | CF₃ | F | F | |
| n-C₆H₁₃ | CF₃ | F | F | |

## Patentansprüche

1. Tetrahydropyran-Verbindungen der Formel I, 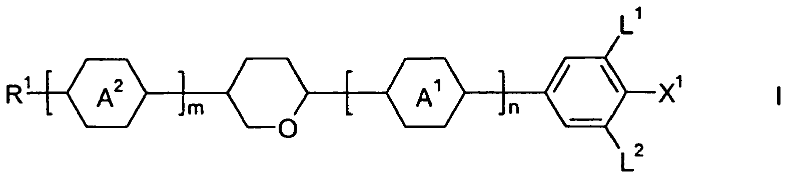 worin
R¹ und X¹ H, einen unsubstituierten oder mit Halogen einfach oder mehrfach substituierten Alkyl- oder Alkoxyrest mit 1 bis 15 Kohlenstoffatomen oder Alkenyl- oder Alkenyloxyrest mit 2 bis 15 Kohlenstoffatomen bedeutet, wobei in diesen Resten auch eine oder mehrere CH₂-Gruppen jeweils unabhängig voneinander durch -C≡C-, -CH=CH-, -CF=CF-, -O-, -(CO)O- oder -O(CO)- so ersetzt sein können, dass O-Atome nicht direkt miteinander verknüpft sind; oder einer der Reste R¹ und X¹ auch F, Cl, CN, NCS oder SF₅,
Ring A¹ und A² unabhängig voneinander 1,4-Cyclohexylen, 1,4-Cyclohexenylen oder 1,4-Cyclohexadienylen, welche unsubstituiert oder durch 1 bis 3 F substituiert sind,
L¹ und L² unabhängig voneinander H oder F,
m 0 oder 1, und
n 1, 2 oder 3,
bedeuten, wobei
m + n ≥2 ist.

2. Verbindungen nach Anspruch 1, **dadurch gekennzeichnet, dass** die Ringe A¹ und A² unabhängig voneinander 1,4-Cyclohexylen oder 1,4-Cyclohexenylen bedeuten.

3. Verbindungen nach Anspruch 1 oder 2, **dadurch gekennzeichnet, dass**
n 1 oder 2
bedeutet.

4. Verbindungen nach einem oder mehreren der Ansprüche 1 bis 3, **dadurch gekennzeichnet, dass**
R¹ Alkyl, Alkoxy, Alkenyl oder Alkenyloxy mit bis zu 8 Kohlenstoffatomen bedeutet.

5. Verbindungen nach einem oder mehreren der Ansprüche 1 bis 4, **dadurch gekennzeichnet, dass**
X¹ F, Cl, CN, NCS, SF₅, einen halogenierten Alkylrest, halogenierten Alkoxyrest, halogenierten Alkenylrest oder halogenierten Alkenyloxyrest mit jeweils bis zu 7 C-Atomen bedeutet.

6. Verbindungen nach einem oder mehreren der Ansprüche 1 bis 5, **dadurch gekennzeichnet, dass**
L¹ Fluor und
L² Fluor oder Wasserstoff bedeuten.

7. Verbindungen nach einem oder mehreren der Ansprüche 1 bis 6, **dadurch gekennzeichnet, dass** L¹ und L² Fluor bedeuten.

8. Verbindungen nach einem oder mehreren der Ansprüche 1 bis 7 der Formeln I1 bis I30, 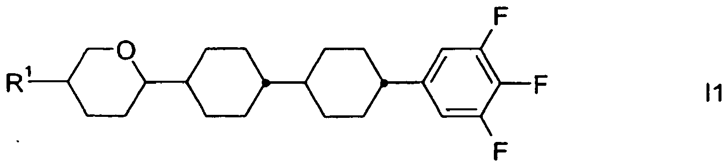 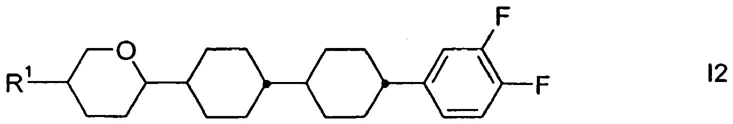 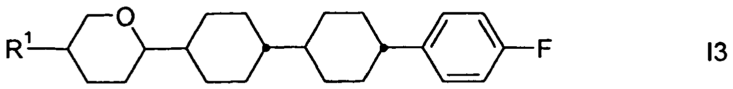 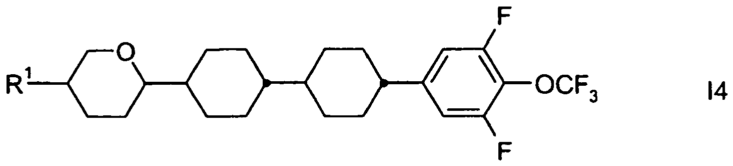 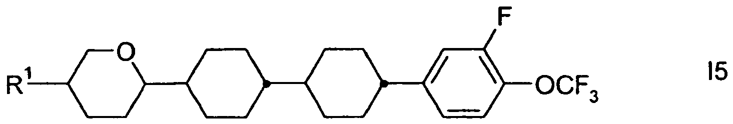 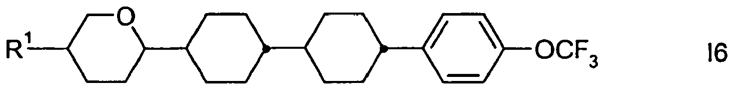 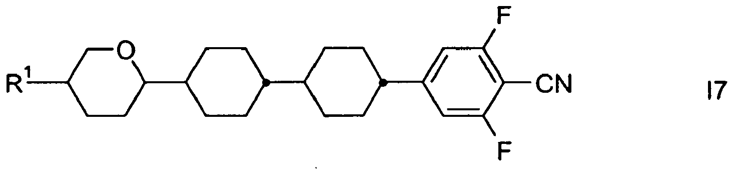 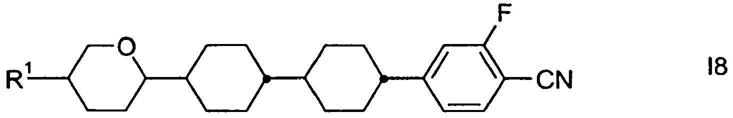 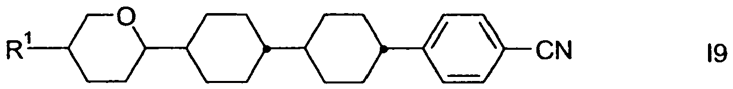 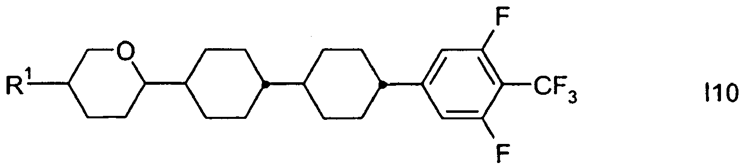 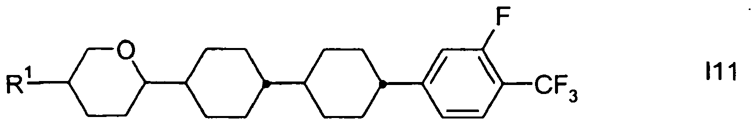 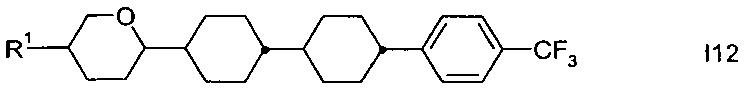 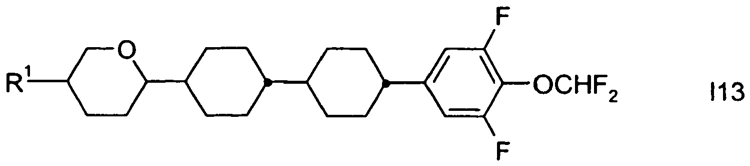 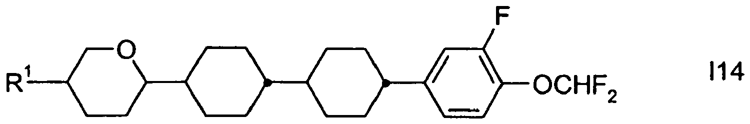 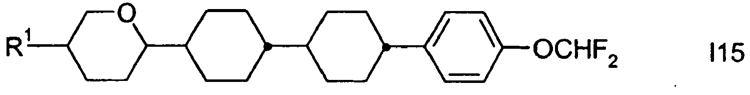 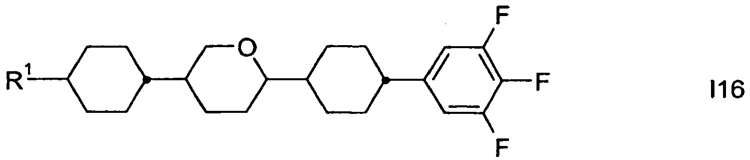 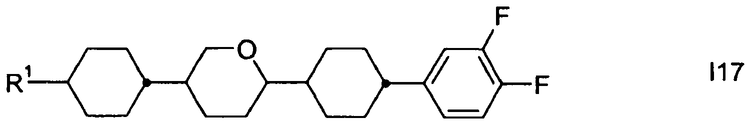 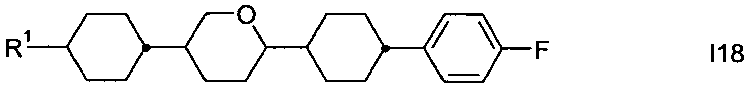 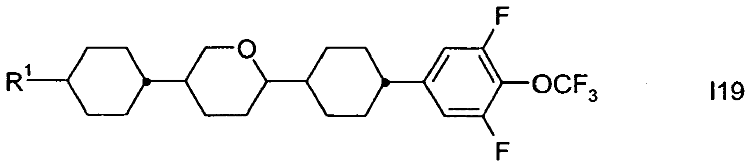 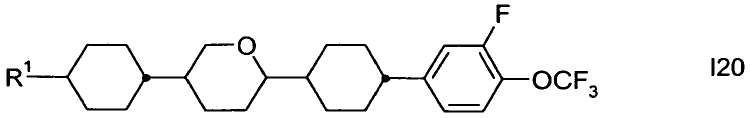 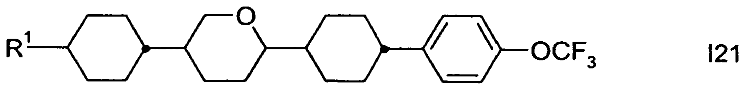 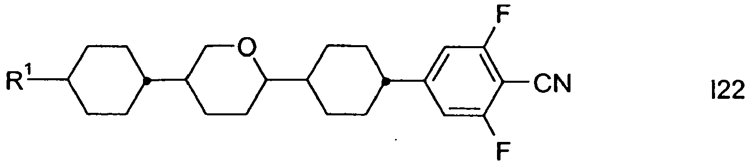 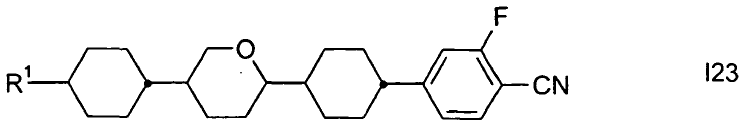 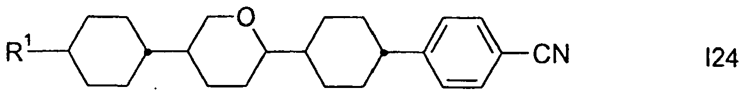 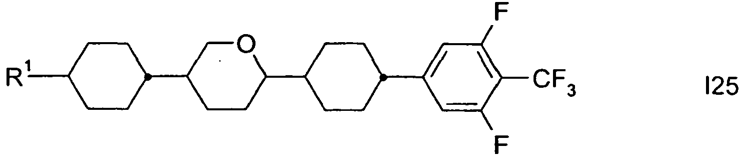 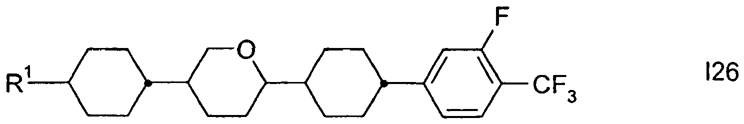 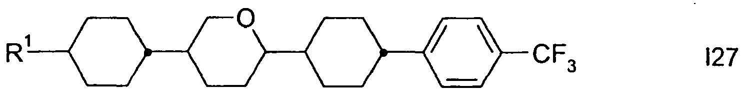 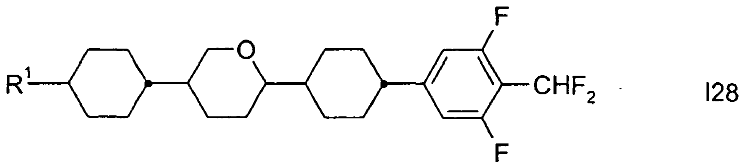 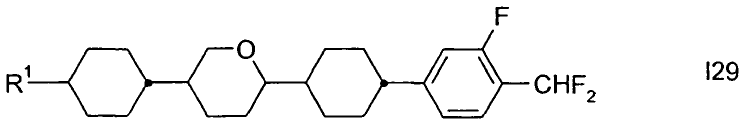 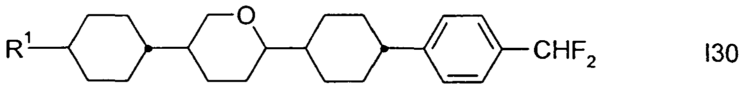 wobei R¹ wie in Anspruch 1 definiert ist.

9. Verbindungen nach einem oder mehreren der Ansprüche 1 bis 8, **dadurch gekennzeichnet, dass** die Substituenten am Tetrahydropyranring in 1,4-Position in trans-Stellung zueinander stehen.

10. Verfahren zur Herstellung von Verbindungen der Formel I nach einem oder mehreren der Ansprüche 1 bis 9 umfassend einen Verfahrensschritt, der **dadurch gekennzeichnet ist, dass** ein Cyclohexanon der Formel II 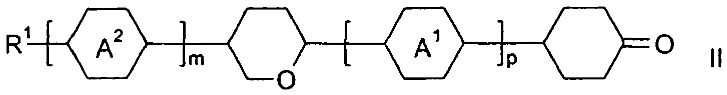 worin
R¹, A¹, A² und m wie in Formel I definiert sind, und
p 0,1 oder 2 bedeutet,
mit einer Arylmetallverbindung der Formel III 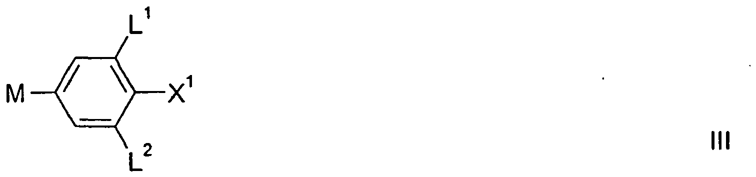 worin
L¹, L² und X¹ wie in Formel I definiert sind, und
M Li, MgCl oder MgBr bedeutet,
umgesetzt wird.

11. Verwendung einer oder mehrerer Verbindungen der Formel I nach einem oder mehreren der Ansprüche 1 bis 9 als Komponenten in einem flüssigkristallinen Medium.

12. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 9 enthält.

13. Verwendung des flüssigkristallinen Mediums nach Anspruch 12 für elektrooptische Zwecke.

14. Elektrooptische Flüssigkristallanzeige enthaltend ein flüssigkristallines Medium nach Anspruch 12.

## Claims

1. Tetrahydropyran compounds of the formula I, 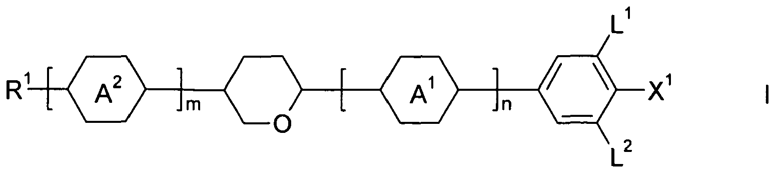 in which
R¹ and X¹ denote H, an unsubstituted or mono- or poly-halogen-substituted alkyl or alkoxy radical having 1 to 15 carbon atoms or alkenyl or alkenyloxy radical having 2 to 15 carbon atoms, where, in addition, one or more CH₂ groups in these radicals may each be replaced, independently of one another, by -C≡C-, -CH=CH-, -CF=CF- , -O-, -(CO)O- or -O(CO)- in such a way that O atoms are not linked directly to one another; or one of the radicals R¹ and X¹ also denotes F, Cl, CN, NCS or SF₅,
rings A¹ and A², independently of one another, denote 1,4-cyclohexylene, 1,4-cyclohexenylene or 1,4-cyclohexadienylene, which are unsubstituted or substituted by 1 to 3 F,
L¹ and L², independently of one another, denote H or F,
m denotes 0 or 1, and
n denotes 1, 2 or 3,
where
m+n is ≥ 2.

2. Compounds according to Claim 1, **characterised in that** the rings A¹ and A², independently of one another, denote 1,4-cyclohexylene or 1,4-cyclohexenylene.

3. Compounds according to Claim 1 or 2, **characterised in that**
n denotes 1 or 2.

4. Compounds according to one or more of Claims 1 to 3, **characterised in that**
R¹ denotes alkyl, alkoxy, alkenyl or alkenyloxy having up to 8 carbon atoms.

5. Compounds according to one or more of Claims 1 to 4, **characterised in that**
x¹ denotes F, Cl, CN, NCS, SF₅, a halogenated alkyl radical, halogenated alkoxy radical, halogenated alkenyl radical or halogenated alkenyloxy radical, each having up to 7 C atoms.

6. Compounds according to one or more of Claims 1 to 5, **characterised in that**
L¹ denotes fluorine and
L² denotes fluorine or hydrogen.

7. Compounds according to one or more of Claims 1 to 6, **characterised in that** L¹ and L² denote fluorine.

8. Compounds according to one or more of Claims 1 to 7 of the formulae I1 to I30, 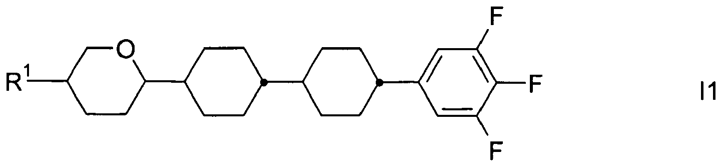 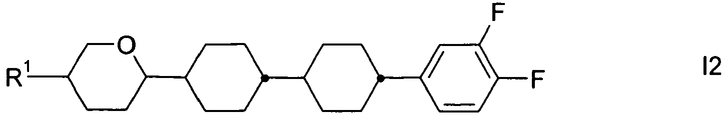 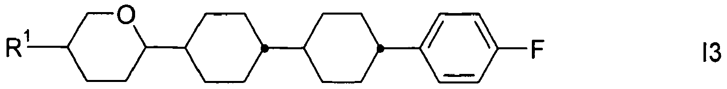 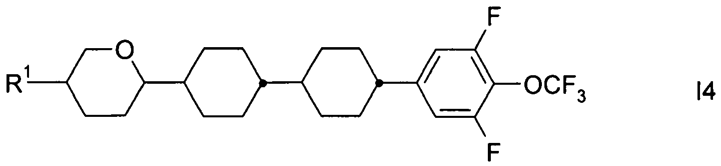 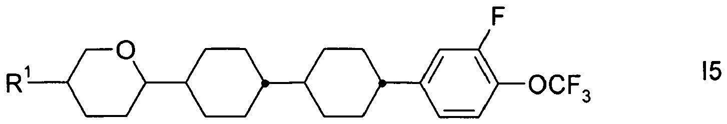 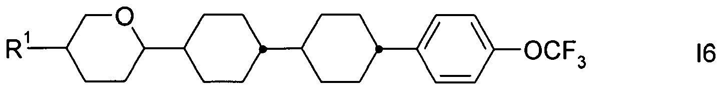 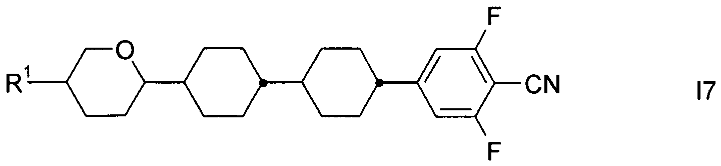 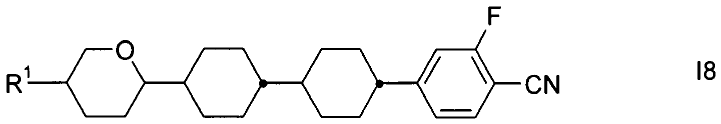 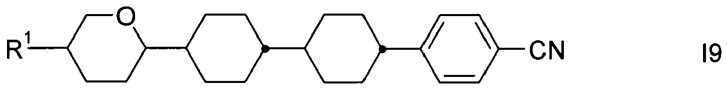 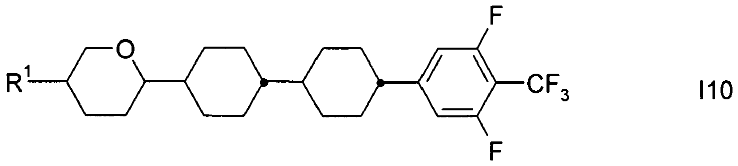 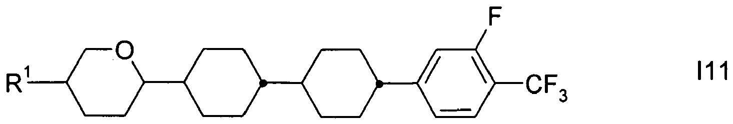 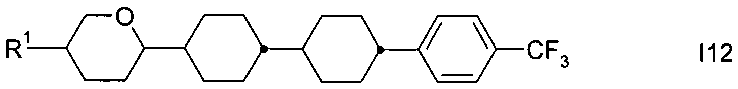 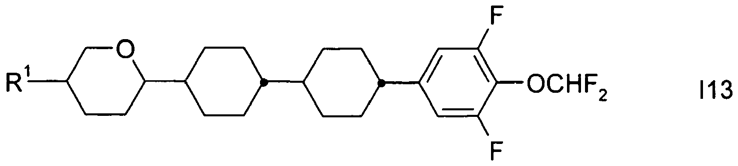 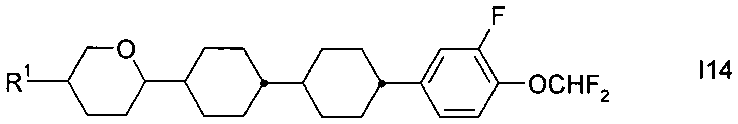 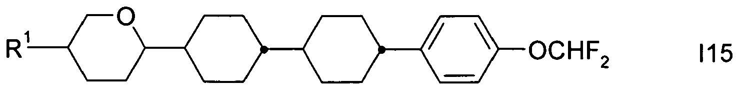 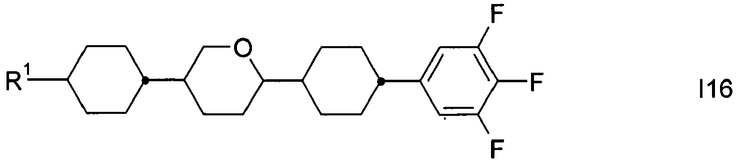 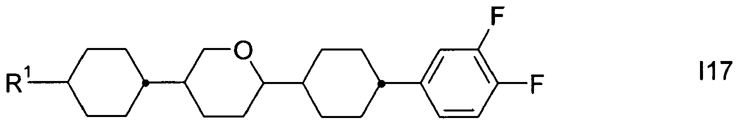 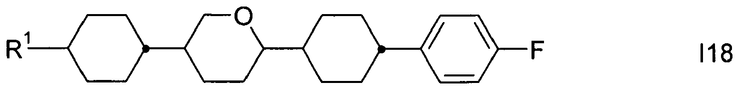 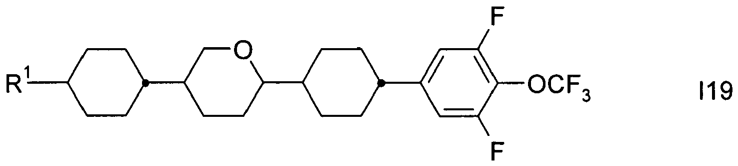 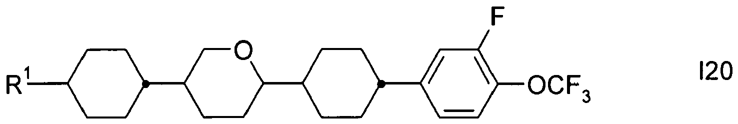 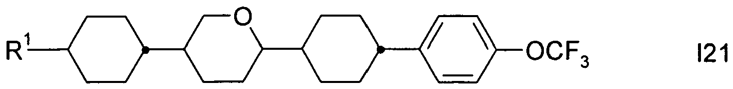 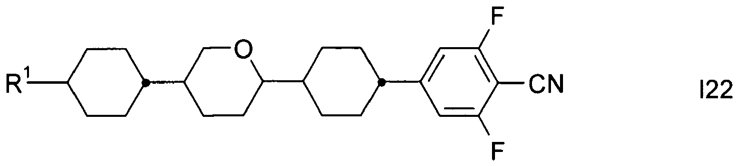 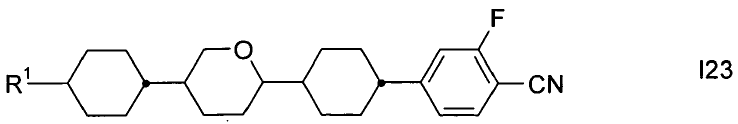 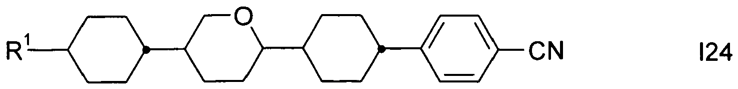 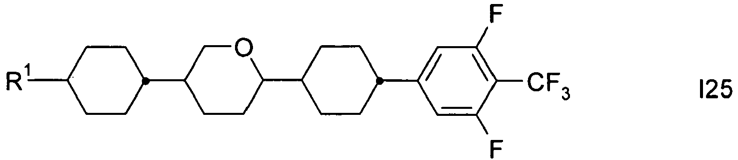 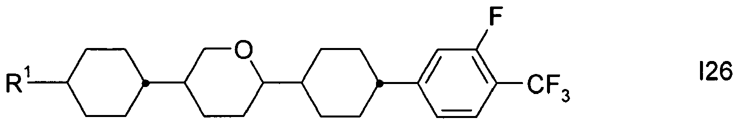 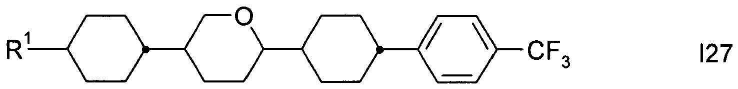 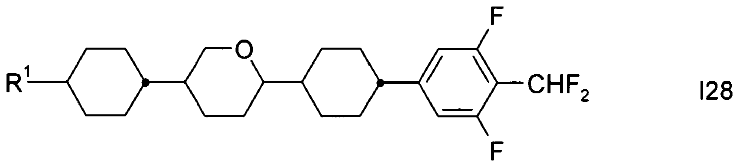 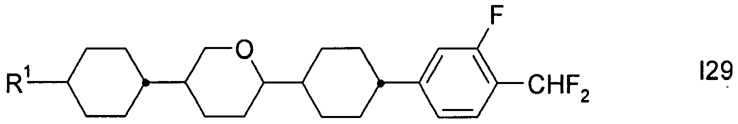 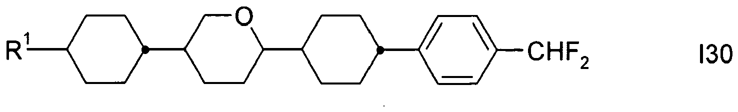 where R¹ is as defined in Claim 1.

9. Compounds according to one or more of Claims 1 to 8, **characterised in that** the substituents on the tetrahydropyran ring in the 1,4-position are in the *trans*-position to one another.

10. Process for the preparation of compounds of the formula I according to one or more of Claims 1 to 9 comprising a process step which is **characterised in that** a cyclohexanone of the formula II 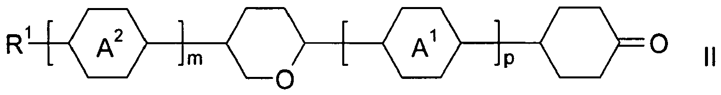 in which
R¹, A¹, A² and m are as defined in formula I, and
p denotes 0, 1 or 2,
is reacted with an arylmetal compound of the formula III 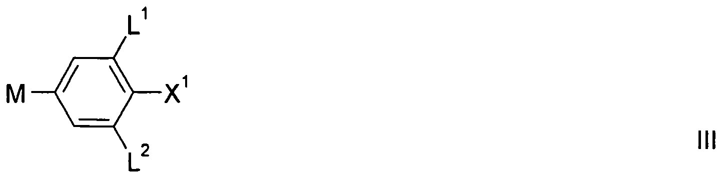 in which
L¹, L² and X¹ are as defined in formula I, and
M denotes Li, MgCI or MgBr.

11. Use of one or more compounds of the formula I according to one or more of Claims 1 to 9 as components in a liquid-crystalline medium.

12. Liquid-crystalline medium comprising at least two mesogenic compounds, **characterised in that** it comprises at least one compound of the formula I according to one or more of Claims 1 to 9.

13. Use of the liquid-crystalline medium according to Claim 12 for electro-optical purposes.

14. Electro-optical liquid-crystal display containing a liquid-crystalline medium according to Claim 12.

## Revendications

1. Composés de tétrahydropyrane de la formule 1, 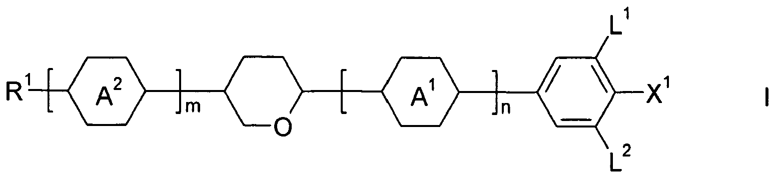 dans laquelle
R¹ et X¹ représentent H, un radical alkyle ou alcoxy non substitué ou mono- ou polysubstitué par halogène comportant de 1 à 15 atomes de carbone ou un radical alkényle ou alkényloxy comportant de 2 à 15 atomes de carbone, où, en outre, un ou plusieurs groupes CH₂ dans ces radicaux peuvent chacun être remplacés, indépendamment les uns des autres, par -C≡C-, -CH=CH-, -CF=CF-, -O-, -(CO)O- ou -O(CO)- de telle sorte que des atomes de O ne soient pas liés directement les uns aux autres; ou l'un des radicaux R¹ et X¹ représente également F, CI, CN, NCS ou SF₅,
les cycles A¹ et A², indépendamment l'un de l'autre, représentent 1,4-cyclohexylène, 1,4-cyclohexénylène ou 1,4-cyclohexadiénylène, lesquels sont non substitués ou substitués par 1 à 3 F,
L¹ et L², indépendamment l'une l'autre, représentent H ou F,
m représente 0 ou 1, et
n représente 1, 2 ou 3,
où
m+n est ≥ 2.

2. Composés selon la revendication 1, **caractérisés en ce que** les cycles A¹ et A², indépendamment l'un de l'autre, représentent 1,4-cyclohexylène ou 1,4-cyclohexénylène.

3. Composés selon la revendication 1 ou 2, **caractérisés en ce que**
n représente 1 ou 2.

4. Composés selon une ou plusieurs des revendications 1 à 3, **caractérisés en ce que**
R¹ représente alkyle, alcoxy, alkényle ou alkényloxy comportant jusqu'à 8 atomes de carbone.

5. Composés selon une ou plusieurs des revendications 1 à 4, **caractérisés en ce que**
X¹ représente F, CI, CN, NCS, SF₅, un radical alkyle halogéné, un radical alcoxy halogéné, un radical alkényle halogéné ou un radical alkényloxy halogéné, chacun comportant jusqu'à 7 atomes de C.

6. Composés selon une ou plusieurs des revendications 1 à 5, **caractérisés en ce que**
L¹ représente fluor et
L² représente fluor ou hydrogène.

7. Composés selon une ou plusieurs des revendications 1 à 6, **caractérisés en ce que** L¹ et L² représentent fluor.

8. Composés selon une ou plusieurs des revendications 1 à 7 des formules I1 à I30, 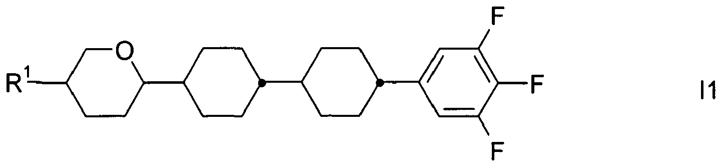 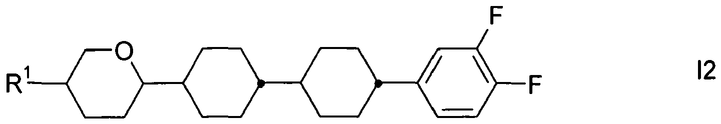 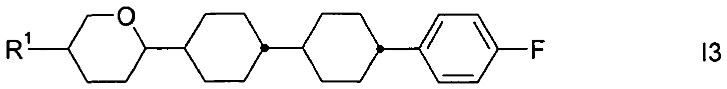 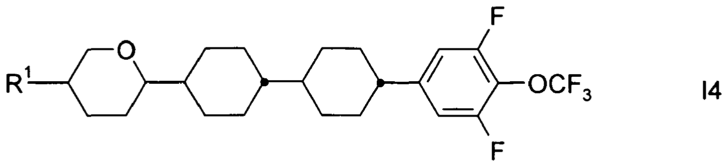 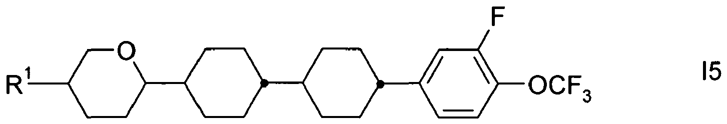 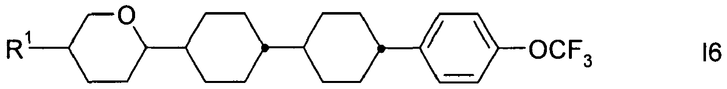 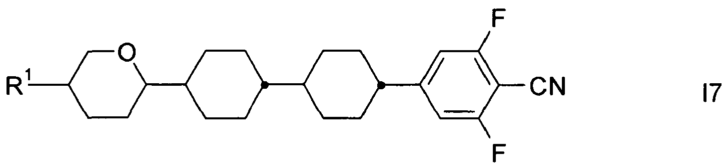 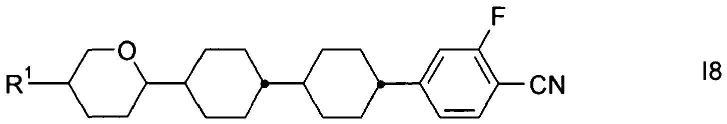 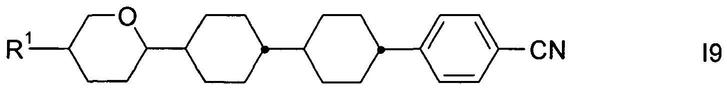 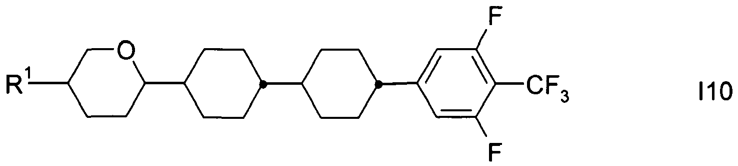 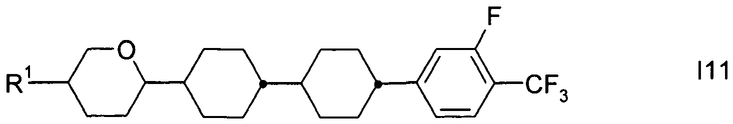 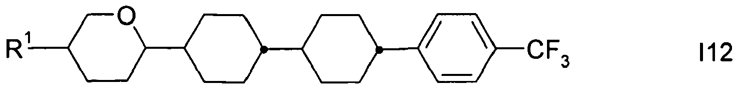 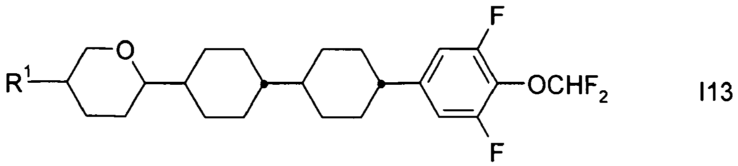 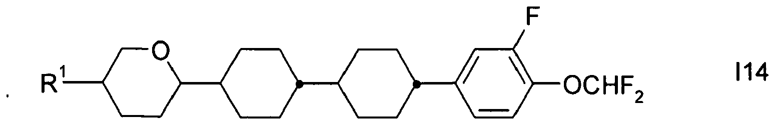 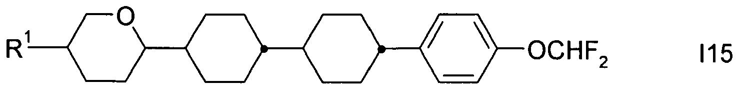 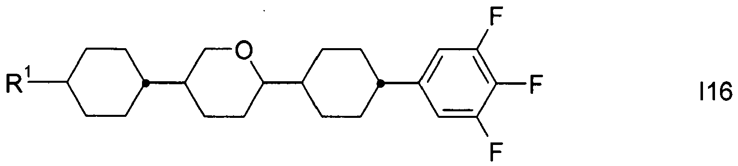 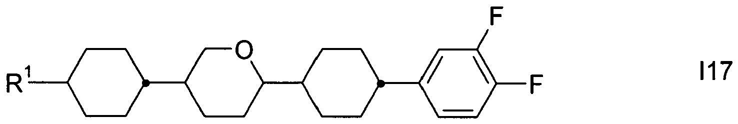 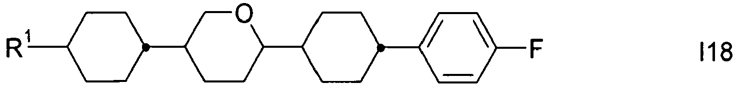 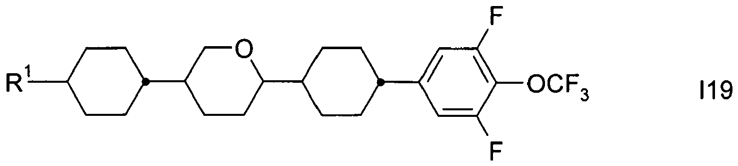 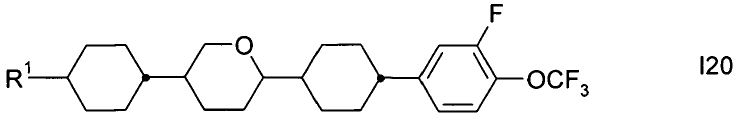 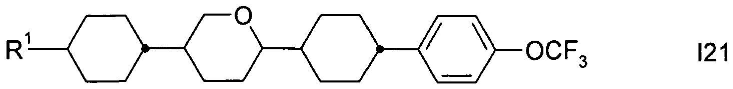 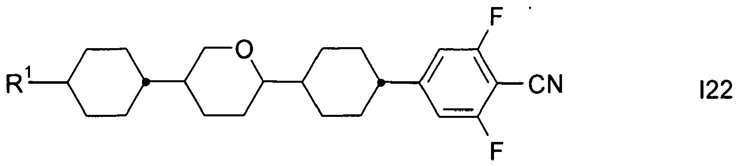 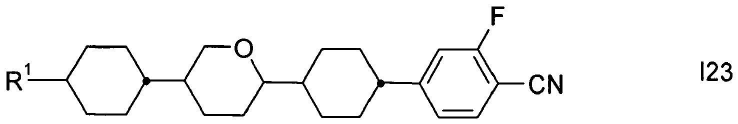 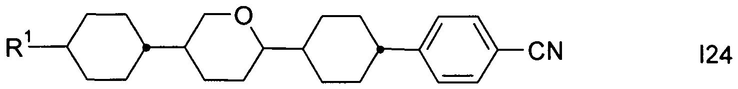 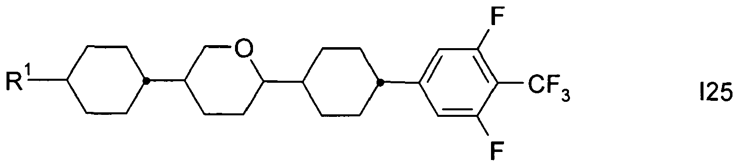 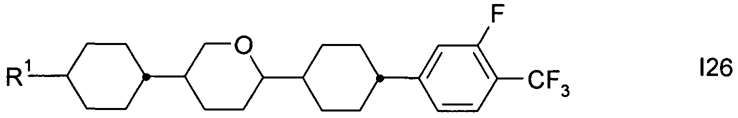 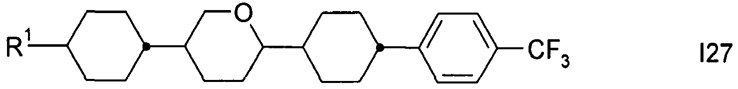 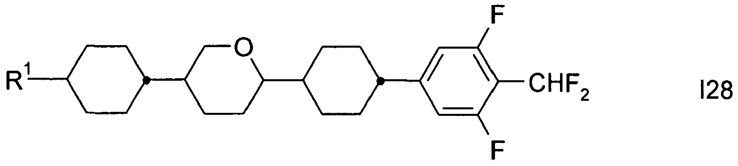 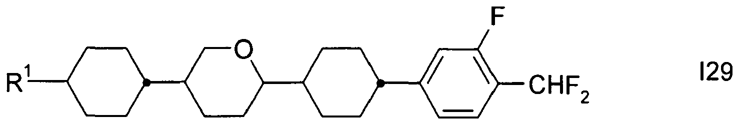 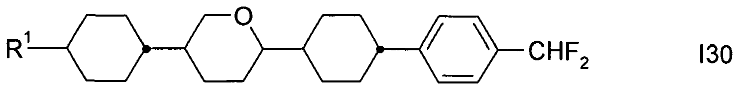 où R¹ est comme défini selon la revendication 1.

9. Composés selon une ou plusieurs des revendications 1 à 8, **caractérisés en ce que** les substituents sur le cycle tétrahydropyrane à la position 1,4 sont à la position *trans* les uns par rapport aux autres.

10. Procédé pour la préparation de composés de la formule I selon une ou plusieurs des revendications 1 à 9 comprenant une étape de procédé qui est **caractérisée en ce qu'**un cyclohexanone de la formule II 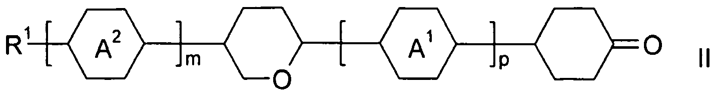 dans laquelle
R¹, A¹, A² et m sont comme défini selon la formule I, et
p représente 0, 1 ou 2,
est amené à réagir avec un composé arylmétal de la formule III 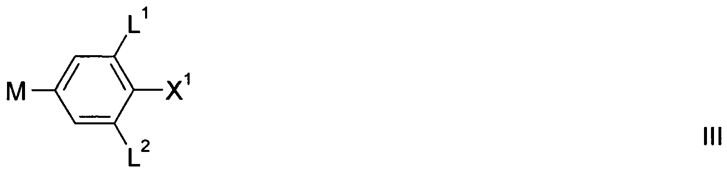 dans laquelle
L¹, L² et X¹ sont comme défini selon la formule I, et
M représente Li, MgCl ou MgBr.

11. Utilisation d'un ou de plusieurs composés de la formule I selon une ou plusieurs des revendications 1 à 9 en tant que composants dans un milieu cristallin liquide.

12. Milieu cristallin liquide comprenant au moins deux composés mésogènes, **caractérisé en ce qu'**il comprend au moins un composé de la formule I selon une ou plusieurs des revendications 1 à 9.

13. Utilisation du milieu cristallin liquide selon la revendication 12 à des fins électro-optiques.

14. Affichage à cristaux liquides électro-optique contenant un milieu cristallin liquide selon la revendication 12.
